# EUROPEAN PATENT SPECIFICATION

(11) **EP 4 546 339 B1**
(45) Date of publication and mention of the grant of the patent: **02.12.2026**
(21) Application number: 24209028.0
(22) Date of filing: 25.10.2024
(51) Int. Cl.: G10L 25/66, A61B 5/00, G16H 50/20, G16H 50/30

(54) **TECHNIQUES FOR SPEECH LANGUAGE MODEL TRAINING AND APPLICATION**
VERFAHREN ZUM TRAINIEREN UND ZUR ANWENDUNG EINES SPRACHMODELLS
TECHNIQUES D'APPRENTISSAGE ET D'APPLICATION DE MODÈLE DE LANGAGE VOCAL

(30) Priority: 26.10.2023 US 202318495588
(43) Date of publication of application: 30.04.2025
(73) Proprietor: Canary Speech, Inc., Lehi, UT 84043 (US); National Cerebral and Cardiovascular Center, Suita 564-8565 (JP); SMK Corporation, Tokyo 142-0041 (JP)
(72) Inventor: KWON, Namhee, Lehi, 84043 (US); BRUECKNER, Raymond, Lehi, 84043 (US); SUBRAMANIAN, Vinod, Lehi, 84043 (US); BLAYLOCK, Nate, Lehi, 84043 (US); O'CONNELL, Henry, Lehi, 84043 (US); NISHIMURA, Kunihiro, Suita, 564-8565 (JP); OGATA, Soshiro, Suita, 564-8565 (JP); KIYOSHIGE, Eri, Suita, 564-8565 (JP); TAKEGAMI, Misa, Suita, 564-8565 (JP)
(74) Representative: Page White Farrer

(56) References cited:
- V?SQUEZ-CORREA JUAN CAMILO ET AL: "Convolutional Neural Networks and a Transfer Learning Strategy to Classify Parkinson's Disease from Speech in Three Different Languages", 22 October 2019, 20191022, PAGE(S) 697 - 706, XP047525427
- RIOS-URREGO CRISTIAN DAVID ET AL: "Transfer Learning to Detect Parkinson's Disease from Speech In Different Languages Using Convolutional Neural Networks with Layer Freezing", 1 September 2020, 20200901, PAGE(S) 331 - 339, XP047560332
- V?SQUEZ-CORREA JUAN CAMILO ET AL: "Transfer learning helps to improve the accuracy to classify patients with different speech disorders in different languages", PATTERN RECOGNITION LETTERS, ELSEVIER, AMSTERDAM, NL, vol. 150, 18 April 2021 (2021-04-18), pages 272 - 279, XP086766901, ISSN: 0167-8655, [retrieved on 20210418], DOI: 10.1016/J.PATREC.2021.04.011
- MANCIOPPI GIANMARIA ET AL: "The use of Motor and Cognitive Dual-Task quantitative assessment on subjects with mild cognitive impairment: A systematic review", MECHANISMS OF AGEING AND DEVELOPMENT, ELSEVIER SEQUOIA, LAUSANNE, CH, vol. 193, 11 November 2020 (2020-11-11), XP086430770, ISSN: 0047-6374, [retrieved on 20201111], DOI: 10.1016/J.MAD.2020.111393
- WANG DONG ET AL: "Transfer learning for speech and language processing", 2015 ASIA-PACIFIC SIGNAL AND INFORMATION PROCESSING ASSOCIATION ANNUAL SUMMIT AND CONFERENCE (APSIPA), ASIA-PACIFIC SIGNAL AND INFORMATION PROCESSING ASSOCIATION, 16 December 2015 (2015-12-16), pages 1225 - 1237, XP032870792, DOI: 10.1109/APSIPA.2015.7415532
- WEIL RIMONA S ET AL: "Mild Cognitive Impairment in Parkinson's Disease-What Is It?", CURRENT NEUROLOGY AND NEUROSCIENCE REPORTS, SPRINGER US, NEW YORK, vol. 18, no. 4, 10 March 2018 (2018-03-10), pages 1 - 11, XP036463087, ISSN: 1528-4042, [retrieved on 20180310], DOI: 10.1007/S11910-018-0823-9
- CALZÀ LAURA ET AL: "Linguistic features and automatic classifiers for identifying mild cognitive impairment and dementia", COMPUTER SPEECH AND LANGUAGE. ELSEVIER, LONDON., GB, vol. 65, 29 June 2020 (2020-06-29), XP086261008, ISSN: 0885-2308, [retrieved on 20200629], DOI: 10.1016/J.CSL.2020.101113
- CHAU HONG-HAN HANK ET AL: "MCI Detection Based on Deep Learning with Voice Spectrogram", 2022 IEEE 4TH EURASIA CONFERENCE ON BIOMEDICAL ENGINEERING, HEALTHCARE AND SUSTAINABILITY (ECBIOS) IEEE, 27 May 2022 (2022-05-27), pages 212 - 216, XP034229850, [retrieved on 20221117], DOI: 10.1109/ECBIOS54627.2022.9945032
- "Automatic Speech Classifier for Mild Cognitive Impairment and Early Dementia", ACM TRANSACTIONS ON COMPUTING FOR HEALTHCARE (HEALTH) ACMPUB27, NEW YORK, NY, vol. 3, no. 1, 15 October 2021 (2021-10-15), pages 1 - 11, XP058980189, ISSN: 2691-1957, DOI: 10.1145/3469089

## Description

### FIELD

This invention relates to voice analysis and more particularly relates to the automated assessment and diagnosis of one or more medical conditions based on collected voice samples.

### BACKGROUND

Assessment of neurological injuries and illnesses and other medical conditions is often performed manually by a medical professional and can be based on a form filled out by hand with pencil and paper. However, manual assessment can be inaccurate and/or inconsistent.

Calzà, Laura et al.: "Linguistic features and automatic classifiers for identifying mild cognitive impairment and dementia", COMPUTER SPEECH AND LANGUAGE. ELSEVIER, LONDON, GB, vol. 65, 29 June 2020, ISSN: 0885-2308, DOI: 10.1016/J.CSL.2020.101113, provides an overview of the use of machine-learning techniques for assessment of various diseases and proposes an automatic classifier for the assessment of mild cognitive impairment (MCI).

Vásquez-Correa Juan Camilo et al.: "Convolutional Neural Networks and a Transfer Learning Strategy to Classify Parkinson's Disease from Speech in Three Different Languages", 22 October 2019, pages 697 - 706, provides an example of assessment of the Parkinson's disease using neural networks and a transfer learning strategy to cover different languages.

### SUMMARY

Apparatuses and methods are presented for techniques for speech language model training and application. The invention is defined by the independent claims.

### BRIEF DESCRIPTION OF THE DRAWINGS

In order that the advantages of the invention will be readily understood, a more particular description of the invention briefly described above will be rendered by reference to specific embodiments that are illustrated in the appended drawings. Understanding that these drawings depict only typical embodiments of the invention and are not therefore to be considered to be limiting of its scope, the invention will be described and explained with additional specificity and detail through the use of the accompanying drawings, in which:
Figure 1A is a schematic block diagram illustrating one embodiment of a system for techniques for speech language model training and application;
Figure 1B is a schematic block diagram illustrating a further embodiment of a system for techniques for speech language model training and application;
Figure 2 is a schematic block diagram illustrating one embodiment of a system for processing speech data with a mathematical model to perform a medical diagnosis;
Figure 3 is a schematic block diagram illustrating one embodiment of a training corpus of speech data;
Figure 4 is a schematic block diagram illustrating one embodiment of a list of prompts for using in diagnosing a medical condition;
Figure 5 is a schematic block diagram illustrating one embodiment of a system for selecting features for training a mathematical model for diagnosing a medical condition;
Figure 6A is a schematic block diagram illustrating one embodiment of a graph of pairs of feature values and diagnosis values;
Figure 6B is a schematic block diagram illustrating a further embodiment of a graph of pairs of feature values and diagnosis values;
Figure 7 is a schematic flowchart diagram illustrating one embodiment of a method for selecting features for training a mathematical model for diagnosing a medical condition;
Figure 8 is a schematic flowchart diagram illustrating one embodiment of a method for selecting prompts for use with a mathematical model for diagnosing a medical condition;
Figure 9 is a schematic flowchart diagram illustrating one embodiment of a method for training a mathematical model for diagnosing a medical condition that is adapted to a set of selected prompts;
Figure 10 is a schematic block diagram illustrating one embodiment of a computing device that may be used to train and deploy a mathematical model for diagnosing a medical condition;
Figure 11 is a schematic block diagram illustrating one embodiment of a diagnostic apparatus;
Figure 12 is a schematic flowchart diagram illustrating one embodiment of a method for techniques for speech language model training and application; and
Figure 13 is a schematic flowchart diagram illustrating a further embodiment of a method for techniques for speech language model training and application.

### DETAILED DESCRIPTION

Generally, the subject matter herein is directed to assessing a user's risk or likelihood of having mild cognitive impairment (MCI) based on speech or voice data analysis using trained machine learning models. As used herein, MCI refers to a small but measurable degree of cognitive decline during the prodromal stage, e.g., between the expected decline of normal aging and the more serious decline of dementia. MCI is not usually detectable by casual conversation or observation, and it is estimated that a majority of people with MCI are unaware that they have a cognitive problem. People with MCI struggle to remember recent conversations or events, keep track of schedules and appointments, or use new guidelines for a task. Distinguishing MCI from normal aging is a difficult task even for the most conscientious primary care physicians, yet doing so is important for timely intervention and optimal treatment results.

As discussed in more detail below, the subject matter described herein is directed to using artificial intelligence, and in particular machine learning, to determine an assessment of MCI in a user using speech data. In particular, a first machine learning model is trained on speech data in a first language, e.g., English, and a second machine learning model is trained for use in a second language, e.g., Japanese, using at least a portion the first machine learning model. In this manner, as described in more detail below, a speech model may be created for analyzing speech data in one language using a speech model that is trained in a different language. The solutions proposed herein may utilize or otherwise be related to solutions described in U.S. Patent No. 10,152,988, U.S. Patent No. 10,311,980, and U.S. Patent Application No. 17/468,288.

As used herein, artificial intelligence (AI) is broadly defined as a branch of computer science dealing in automating intelligent behavior. AI systems may be designed to use machines to emulate and simulate human intelligence and corresponding behavior. This may take many forms, including symbolic or symbol manipulation AI. AI may address analyzing abstract symbols and/or human readable symbols. AI may form abstract connections between data or other information or stimuli. AI may form logical conclusions. AI is the intelligence exhibited by machines, programs, or software. AI has been defined as the study and design of intelligent agents, in which an intelligent agent is a system that perceives its environment and takes actions that maximize its chances of success.

AI may have various attributes such as deduction, reasoning, and problem solving. AI may include knowledge representation or learning. AI systems may perform natural language processing, perception, motion detection, and information manipulation. At higher levels of abstraction, it may result in social intelligence, creativity, and general intelligence. Various approaches are employed including cybernetics and brain simulation, symbolic, sub-symbolic, and statistical, as well as integrating the approaches.

Various AI tools may be employed, either alone or in combinations. The tools may include search and optimization, logic, probabilistic methods for uncertain reasoning, classifiers and statistical learning methods, neural networks, deep feedforward neural networks, deep recurrent neural networks, deep learning, control theory and languages.

Machine learning (ML) plays an important role in a wide range of critical applications with large volumes of data, such as data mining, natural language processing, image recognition, voice recognition and many other intelligent systems. There are some basic common threads about the definition of ML. As used herein, ML is defined as the field of study that gives computers the ability to learn without being explicitly programmed. For example, for predicting traffic patterns at a busy intersection, it is possible to run through a machine learning algorithm/ model with data about past or historical traffic patterns, e.g., to train the machine learning algorithm/model. The program can correctly predict future traffic patterns if it learned/trained correctly from past patterns.

There are different ways an algorithm can model a problem based on its interaction with the experience, environment, or input data. The machine learning algorithms may be categorized so that it helps to think about the roles of the input data and the model preparation process leading to correct selection of the most appropriate category for a problem to get the best result. Known categories are supervised learning, unsupervised learning, semi-supervised learning, and reinforcement learning.
- (a) In supervised learning category, input data is called training data and has a known label or result. A model is prepared through a training process where it is required to make predictions and is corrected when those predictions are wrong. The training process continues until the model achieves a desired level of accuracy on the training data. Example problems are classification and regression.
- (b) In unsupervised learning category, input data is not labelled and does not have a known result. A model is prepared by deducing structures present in the input data. Example problems are association rule learning and clustering. An example algorithm is k-means clustering.
- (c) Semi-supervised learning falls between unsupervised learning (without any labeled training data) and supervised learning (with completely labeled training data). Researchers found that unlabeled data, when used in conjunction with a small amount of labeled data may produce considerable improvement in learning accuracy.
- (d) Reinforcement learning is another category which differs from standard supervised learning in that correct input/output pairs are never presented. Further, there is a focus on on-line performance, which involves finding a balance between exploration for new knowledge and exploitation of current knowledge already discovered.

Certain machine learning techniques are widely used and are as follows: Decision tree learning, Association rule learning, Artificial neural networks, Inductive logic programming, Support vector machines, Clustering, Bayesian networks, Reinforcement learning, Representation learning, and Genetic algorithms. In certain embodiments, multiple machine learning algorithms may be applied using ensemble learning. As used herein, ensemble learning may refer to a machine learning technique that combines multiple algorithms to produce a single predictive model.

The learning processes in machine learning algorithms are generalizations from past experiences. After having experienced a learning data set, the generalization process is the ability of a machine learning algorithm to accurately execute on new examples and tasks. The learner needs to build a general model about a problem space enabling a machine learning algorithm to produce sufficiently accurate predictions in future cases. The training examples may come from some generally unknown probability distribution.

In theoretical computer science, computational learning theory performs computational analysis of machine learning algorithms and their performance. The training data set is limited in size and may not capture all forms of distributions in future data sets. The performance is represented by probabilistic bounds. Errors in generalization are quantified by bias-variance decompositions. The time complexity and feasibility of learning in computational learning theory describes a computation to be feasible if it is done in polynomial time. Positive results are determined and classified when a certain class of functions can be learned in polynomial time whereas negative results are determined and classified when learning cannot be done in polynomial time.

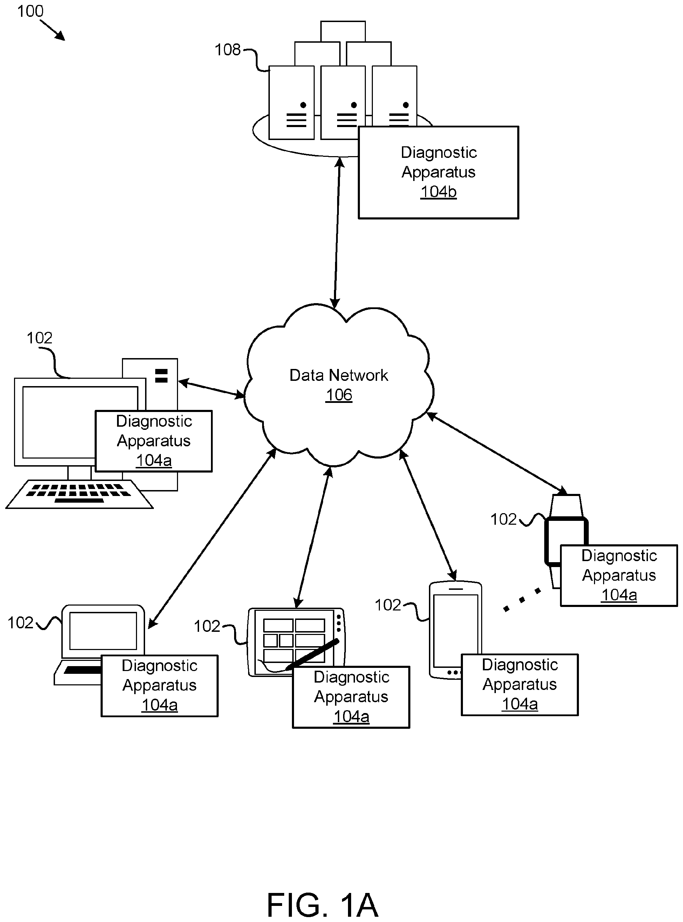

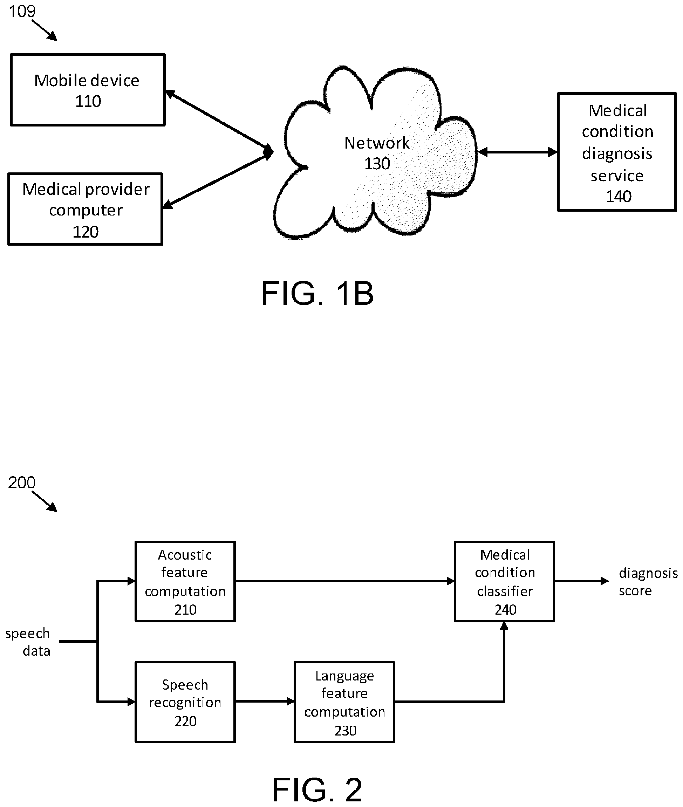

Figure 1A depicts one embodiment of a system 100 for techniques for speech language model training and application. In one embodiment, the system 100 includes one or more hardware devices 102, one or more diagnostic apparatuses 104 (e.g., one or more diagnostic apparatuses 104a disposed on the one or more hardware devices 102, one or more backend diagnostic apparatuses 104b, or the like), one or more data networks 106 or other communication channels, and/or one or more backend servers 108. In certain embodiments, even though a specific number of hardware devices 102, diagnostic apparatuses 104, data networks 106, and/or backend servers 108 are depicted in Figure 1, one of skill in the art will recognize, in light of this disclosure, that any number of hardware devices 102, diagnostic apparatuses 104, data networks 106, and/or backend servers 108 may be included in the system 100 for voice collection and/or techniques for speech language model training and application.

In general, the diagnostic apparatus 104 is configured to train a first speech model in a first language, the first speech model used to determine one or more characteristics of speech data that is indicative of MCI, train a second speech model for use in a second language using at least a portion of the first speech model trained in the first language, apply the second speech model trained for use in the second language to speech data for a user captured in the second language, and determine, based on output from the second speech model trained for use in the second language, an assessment of MCI for the user. The diagnostic apparatus 104, including its various sub-modules, may be located on one or more information handling devices 102 in the system 100, one or more servers 108, one or more network devices, and/or the like. The diagnostic apparatus 104 is described in more detail below.

In one embodiment, the system 100 includes one or more hardware devices 102. The hardware devices 102 and/or the one or more backend servers 108 (e.g., computing devices, information handling devices, or the like) may include one or more of a desktop computer, a laptop computer, a mobile device, a tablet computer, a smart phone, a set-top box, a gaming console, a smart TV, a smart watch, a fitness band, an optical head-mounted display (e.g., a virtual reality headset, smart glasses, or the like), an HDMI or other electronic display dongle, a personal digital assistant, and/or another computing device comprising a processor (e.g., a central processing unit (CPU), a processor core, a field programmable gate array (FPGA) or other programmable logic, an application specific integrated circuit (ASIC), a controller, a microcontroller, and/or another semiconductor integrated circuit device), a volatile memory, and/or a non-volatile storage medium. In certain embodiments, the hardware devices 102 are in communication with one or more backend servers 108 via a data network 106, described below. The hardware devices 102, in a further embodiment, are capable of executing various programs, program code, applications, instructions, functions, or the like.

In various embodiments, a diagnostic apparatus 104 may be embodied as hardware, software, or some combination of hardware and software. In one embodiment, a diagnostic apparatus 104 may comprise executable program code stored on a non-transitory computer readable storage medium for execution on a processor of a hardware device 102; a backend server 108; or the like. For example, a diagnostic apparatus 104 may be embodied as executable program code executing on one or more of a hardware device 102; a backend server 108; a combination of one or more of the foregoing; or the like. In such an embodiment, the various modules that perform the operations of a diagnostic apparatus 104, as described below, may be located on a hardware device 102; a backend server 108; a combination of the two; and/or the like.

In various embodiments, a diagnostic apparatus 104 may be embodied as a hardware appliance that can be installed or deployed on a backend server 108, on a user's hardware device 102 (e.g., a dongle, a protective case for a phone 102 or tablet 102 that includes one or more semiconductor integrated circuit devices within the case in communication with the phone 102 or tablet 102 wirelessly and/or over a data port such as USB or a proprietary communications port, or another peripheral device), or elsewhere on the data network 106 and/or collocated with a user's hardware device 102. In certain embodiments, a diagnostic apparatus 104 may comprise a hardware device such as a secure hardware dongle or other hardware appliance device (e.g., a set-top box, a network appliance, or the like) that attaches to another hardware device 102, such as a laptop computer, a server, a tablet computer, a smart phone, or the like, either by a wired connection (e.g., a USB connection) or a wireless connection (e.g., Bluetooth^{®}, Wi-Fi^{®}, near-field communication (NFC), or the like); that attaches to an electronic display device (e.g., a television or monitor using an HDMI port, a DisplayPort port, a Mini DisplayPort port, VGA port, DVI port, or the like); that operates substantially independently on a data network 106; or the like. A hardware appliance of a diagnostic apparatus 104 may comprise a power interface, a wired and/or wireless network interface, a graphical interface (e.g., a graphics card and/or GPU with one or more display ports) that outputs to a display device, and/or a semiconductor integrated circuit device as described below, configured to perform the functions described herein with regard to a diagnostic apparatus 104.

A diagnostic apparatus 104, in such an embodiment, may comprise a semiconductor integrated circuit device (e.g., one or more chips, die, or other discrete logic hardware), or the like, such as a field-programmable gate array (FPGA) or other programmable logic, firmware for an FPGA or other programmable logic, microcode for execution on a microcontroller, an application-specific integrated circuit (ASIC), a processor, a processor core, or the like. In one embodiment, a diagnostic apparatus 104 may be mounted on a printed circuit board with one or more electrical lines or connections (e.g., to volatile memory, a non-volatile storage medium, a network interface, a peripheral device, a graphical/display interface. The hardware appliance may include one or more pins, pads, or other electrical connections configured to send and receive data (e.g., in communication with one or more electrical lines of a printed circuit board or the like), and one or more hardware circuits and/or other electrical circuits configured to perform various functions of a diagnostic apparatus 104.

The semiconductor integrated circuit device or other hardware appliance of a diagnostic apparatus 104, in certain embodiments, comprises and/or is communicatively coupled to one or more volatile memory media, which may include but is not limited to: random access memory (RAM), dynamic RAM (DRAM), cache, or the like. In one embodiment, the semiconductor integrated circuit device or other hardware appliance of a diagnostic apparatus 104 comprises and/or is communicatively coupled to one or more non-volatile memory media, which may include but is not limited to: NAND flash memory, NOR flash memory, nano random access memory (nano RAM or NRAM), nanocrystal wire-based memory, silicon-oxide based sub-10 nanometer process memory, graphene memory, Silicon-Oxide-Nitride-Oxide-Silicon (SONOS), resistive RAM (RRAM), programmable metallization cell (PMC), conductive-bridging RAM (CBRAM), magneto-resistive RAM (MRAM), dynamic RAM (DRAM), phase change RAM (PRAM or PCM), magnetic storage media (e.g., hard disk, tape), optical storage media, or the like.

The data network 106, in one embodiment, includes a digital communication network that transmits digital communications. The data network 106 may include a wireless network, such as a wireless cellular network, a local wireless network, such as a Wi-Fi network, a Bluetooth^{®} network, a near-field communication (NFC) network, an ad hoc network, and/or the like. The data network 106 may include a wide area network (WAN), a storage area network (SAN), a local area network (LAN), an optical fiber network, the internet, or other digital communication network. The data network 106 may include two or more networks. The data network 106 may include one or more servers, routers, switches, and/or other networking equipment. The data network 106 may also include one or more computer readable storage media, such as a hard disk drive, an optical drive, non-volatile memory, RAM, or the like.

The one or more backend servers 108, in one embodiment, may include one or more network accessible computing systems such as one or more web servers hosting one or more web sites, an enterprise intranet system, an application server, an application programming interface (API) server, an authentication server, or the like. A backend server 108 may include one or more servers located remotely from the hardware devices 102. A backend server 108 may include at least a portion of the diagnostic apparatuses 104, may comprise hardware of a diagnostic apparatus 104, may store executable program code of a diagnostic apparatus 104 in one or more non-transitory computer readable storage media, and/or may otherwise perform one or more of the various operations of a diagnostic apparatus 104 described herein for shared content tracking and attribution.

Figure 1B is an example system 109 for diagnosing a medical condition using a person's speech. Figure 1B includes a medical condition diagnosis service 140 that may receive speech data of a person and process the speech data to determine if a person has a medical condition. For example, medical condition diagnosis service 140 may process the speech data to compute a yes or no determination as to whether the person has the medical condition or to compute a score that indicates a probability or a likelihood that the person has the medical condition and/or a severity of the condition.

As used herein, a diagnosis relates to any determination as to whether a person may have a medical condition or any determination as to a possible severity of the medical condition. A diagnosis may include any form of an assessment, conclusion, opinion, or determination relating to a medical condition. In some instances, a diagnosis may be incorrect, and a person diagnosed with a medical condition may not actually have the medical condition.

Medical condition diagnosis service 140 may receive the speech data of a person using any appropriate techniques. For example, a person may speak to a mobile device 110 and mobile device 110 may record the speech and transmit the recorded speech data to medical condition diagnosis service 140 over network 130. Any appropriate techniques and any appropriate network may be used for mobile device 110 to transmit the recorded speech data to medical condition diagnosis service 140. For example, an application or "app" may be installed on mobile device 110 that uses a REST (representational state transfer) API (application programming interface) call to transmit the speech data over the Internet or a mobile telephone network. In another example, a medical provider may have a medical provider computer 120 that is used to record speech of a person and transmit speech data to medical condition diagnosis service 140.

In some implementations, medical condition diagnosis service 140 may be installed on mobile device 110 or medical provider computer 120 such that it is not necessary to transmit the speech data over a network. The example of Figure 1B is not limiting, and any appropriate techniques may be used to transmit speech data for processing by a mathematical model.

The output of medical condition diagnosis service 140 may then be used for any appropriate purpose. For example, information may be presented to the person who provided the speech data or to a medical professional who is treating the person.

Figure 2 is an example system 200 for processing speech data with a mathematical model to perform a medical diagnosis. **In** processing the speech data, features may be computed from the speech data, and then the features may be processed by the mathematical model. Any appropriate type of features may be used.

The features may include acoustic features, where acoustic features are any features computed from the speech data that do not involve or depend on performing speech recognition on the speech data (e.g., the acoustic features do not use information about the words spoken in the speech data). For example, acoustic features may include mel-frequency cepstral coefficients, perceptual linear prediction features, jitter, or shimmer.

The features may include language features where language features are computed using the results of a speech recognition. For example, language features may include a speaking rate (e.g., the number of vowels or syllables per second), a number of pause fillers (e.g., "ums" and "ahs"), the difficulty of words (e.g., less common words), or the parts of speech of words following pause fillers.

In Figure 2, the speech data is processed by acoustic feature computation component 210 and speech recognition component 220. Acoustic feature computation component 210 may compute acoustic features from the speech data, such as any of the acoustic features described herein. Speech recognition component 220 may perform automatic speech recognition on the speech data using any appropriate techniques (e.g., Gaussian mixture models, acoustic modelling, language modelling, and neural networks).

Because speech recognition component 220 may use acoustic features in performing speech recognition, some processing of these two components may overlap and thus other configurations are possible. For example, acoustic feature component 210 may compute the acoustic features needed by speech recognition component 220, and speech recognition component 220 may thus not need to compute any acoustic features.

Language feature computation component 230 may receive speech recognition results from speech recognition component 220 and process the speech recognition results to determine language features, such as any of the language features described herein. The speech recognition results may be in any appropriate format and include any appropriate information. For example, the speech recognition results may include a word lattice that includes multiple possible sequences of words, information about pause fillers, and the timings of words, syllables, vowels, pause fillers, or any other unit of speech.

Medical condition classifier 240 may process the acoustic features and the language features with a mathematical model to output one or more diagnosis scores that indicate whether the person has the medical condition, such as a score indicating a probability or likelihood that the person has the medical condition and/or a score indicating a severity of the medical condition. Medical condition classifier 240 may use any appropriate techniques, such as a classifier implemented with a support vector machine or a neural network, such as a multi-layer perceptron, e.g., a fully-connected dense network, a convolutional neural network, and/or the like.

The performance of medical condition classifier 240 may depend on the features computed by acoustic feature computation component 210 and language feature computation component 230. Further, a set of features that performs well for one medical condition may not perform well for another medical condition. For example, word difficulty may be an important feature for diagnosing Alzheimer's disease but may not be useful for determining if a person has a concussion. For another example, features relating to the pronunciation of vowels, syllables, or words may be important for Parkinson's disease but may be less important for other medical conditions. Accordingly, techniques are needed for determining a first set of features that performs well for a first medical condition, and this process may need to be repeated for determining a second set of features that performs well for a second medical condition.

In some implementations, medical condition classifier 240 may use other features, which may be referred to as non-speech features, in addition to acoustic features and language features. For example, features may be obtained or computed from demographic information of a person (e.g., gender, age, or place of residence), information from a medical history (e.g., weight, recent blood pressure readings, or previous diagnoses), or any other appropriate information.

The selection of features for diagnosing a medical condition may be more important in situations where an amount of training data for training the mathematical model is relatively small. For example, for training a mathematical model for diagnosing concussions, the needed training data may include speech data of a number of individuals shortly after they experience a concussion. Such data may exist in small quantities and obtaining further examples of such data may take a significant period of time.

Training mathematical models with a smaller amount of training data may result in overfitting where the mathematical model is adapted to the specific training data but because of the small amount of training data, the model may not perform well on new data. For example, the model may be able to detect all of the concussions in the training data but may have a high error rate when processing production data of people who may have concussions.

One technique for preventing overfitting when training a mathematical model is to reduce the number of features used to train the mathematical model. The amount of training data needed to train a model without overfitting increases as the number of features increases. Accordingly, using a smaller number of features allows models to be built with a smaller amount of training data.

Where it is needed to train a model with a smaller number of features, it becomes more important to select the features that will allow the model to perform well. For example, when a large amount of training data is available, hundreds of features may be used to train the model and it is more likely that appropriate features have been used. Conversely, where a small amount of training data is available, only 10 or so features may be used to train a model, and it is more important to select the features that are most important for diagnosing the medical condition.

Now presented are examples of features that may be used to diagnose a medical condition.

Acoustic features may be computed using short-time segment features. When processing speech data, the duration of the speech data may vary. For example, some speech may be a second or two and other speech may be several minutes or more. For consistency in processing speech data, it may be processed in short-time segments (sometimes referred to as frames). For example, each short-time segment may be 25 milliseconds, and segments may advance in increments of 10 milliseconds so that there is a 15 millisecond overlap over two successive segments.

The following are non-limiting examples of short-time segment features: spectral features (such as mel-frequency cepstral coefficients or perceptual linear predictives); prosodic features (such as pitch, energy, or probability of voicing); voice quality features (such as jitter, jitter of jitter, shimmer, or harmonics-to-noise ratio); entropy (e.g., to capture how precisely an utterance is pronounced where entropy may be computed from the posteriors of an acoustic model that is trained on natural speech data).

The short-time segment features may be combined to compute acoustic features for the speech. For example, a two-second speech sample may produce 200 short-time segment features for pitch that may be combined to compute one or more acoustic features for pitch.

The short-time segment features may be combined to compute an acoustic feature for a speech sample using any appropriate techniques. In some implementations, an acoustic feature may be computed using statistics of the short-time segment features (e.g., arithmetic mean, standard deviation, skewness, kurtosis, first quartile, second quartile, third quartile, the second quartile minus the first quartile, the third quartile minus the first quartile, the third quartile minus the second quartile, 0.01 percentile, 0.99 percentile, the 0.99 percentile minus the 0.01 percentile, the percentage of short-time segments whose values are above a threshold (e.g., where the threshold is 75% of the range plus the minimum), the percentage of segments whose values are above a threshold (e.g., where the threshold is 90% of the range plus the minimum), the slope of a linear approximation of the values, the offset of a linear approximation of the values, the linear error computed as the difference of the linear approximation and the actual values, or the quadratic error computed as the difference of the linear approximation and the actual values. In some implementations, an acoustic feature may be computed as a speech embedding to represent the partial or full audio. The speech embedding may include identity vectors such as an i-vector or an x-vector of the short-time segment features and speech representation based on a self-supervised pre-trained model, such as wav2vec or Trillson. An identity vector may be computed using any appropriate techniques, such as performing a matrix-to-vector conversion using a factor analysis technique and a Gaussian mixture model for an i-vector or a neural network model for an x-vector.

The following are non-limiting examples of language features. A speaking rate, such as by computing the duration of all spoken words divided by the number of vowels or any other appropriate measure of speaking rate. A number of pause fillers that may indicate hesitation in speech, such as (1) a number of pause fillers divided by the duration of spoken words or (2) a number of pause fillers divided by the number of spoken words. A measure of word difficulty or the use of less common words. For example, word difficulty may be computed using statistics of 1-gram probabilities of the spoken words, such as by classifying words according to their frequency percentiles (e.g., 5%, 10%, 15%, 20%, 30%, or 40%). The parts of speech of words following pause fillers, such as (1) the counts of each part-of-speech class divided by the number of spoken words or (2) the counts of each part-of-speech class divided by the sum of all part-of-speech counts.

In some implementations, language features may include a determination of whether a person answered a question correctly. For example, a person may be asked what the current year is or who the President of the United States is. The person's speech may be processed to determine what the person said in response to the question and to determine if the person answered the question correctly. Further, in some embodiments, language features may include a determination whether a person read correctly, e.g., read a presented passage correctly. In such an embodiment, the word error rate is computer and compared to the expected reading script, e.g., using an automatic speech recognition (ASR) result. In some embodiments, when the question prompt is intended to assess a verbal fluency test, e.g., asking the user to list the words in a category such as animals, an evaluation is performed to determine if the user's response actually belongs to the expected category by checking or calculating the distance between word vectors.

To train a model for diagnosing a medical condition, a corpus of training data may be collected. The training corpus may include examples of speech where the diagnosis of the person is known. For example, it may be known that the person had no concussion, or a mild, moderate, or severe concussion.

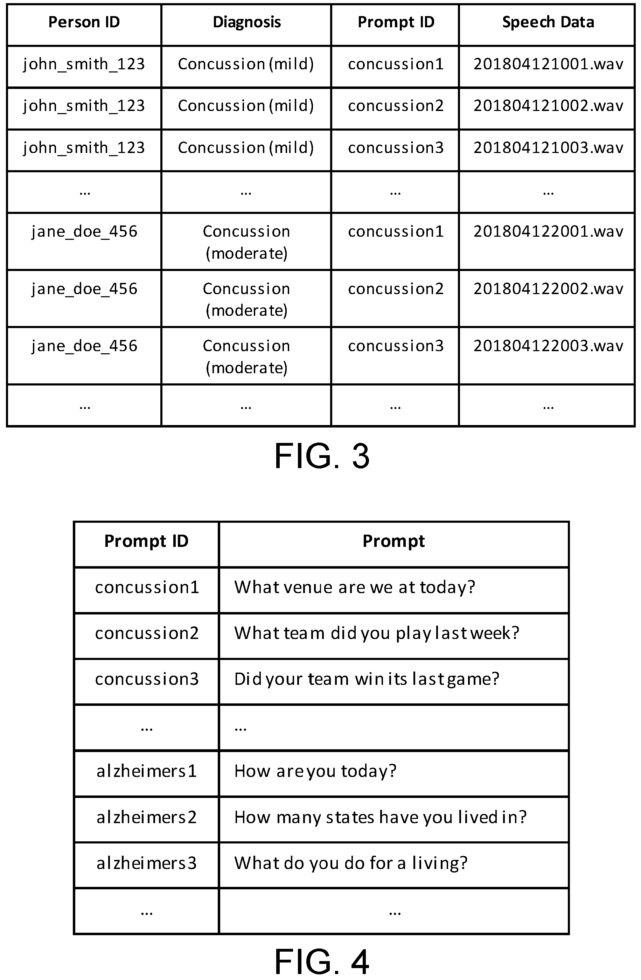

Figure 3 illustrates an example of a training corpus that includes speech data for training a model for diagnosing concussions. For example, the rows of the table of Figure 3 may correspond to database entries. In this example, each entry includes an identifier of a person, the known diagnosis of the person (e.g., no concussion or a mild, medium, or severe concussion), an identifier of a prompt or question that was presented to a person (e.g., "How are you today?"), and a filename of a file that contains the speech data. The training data may be stored in any appropriate format using any appropriate storage technology.

The training corpus may store a representation of a person's speech using any appropriate format. For example, a speech data item of the training corpus may include digital samples of an audio signal received at a microphone or may include a processed version of the audio signal, such as mel-frequency cepstral coefficients.

A single training corpus may contain speech data relating to multiple medical conditions, or a separate training corpus may be used for each medical condition (e.g., a first training corpus for concussions and a second training corpus for Alzheimer's disease). A separate training corpus may be used for storing speech data for people with no known or diagnosed medical condition, as this training corpus may be used for training models for multiple medical conditions.

Figure 4 illustrates an example of stored prompts that may be used to diagnose medical conditions. Each prompt may be presented to a person, either by a person (e.g., a medical professional) or a computer, to obtain speech of the person in response to the prompt. Each prompt may have a prompt identifier so that it may be cross referenced with the prompt identifier of the training corpus. The prompts of Figure 4 may be stored using any appropriate storage technology, such as a database.

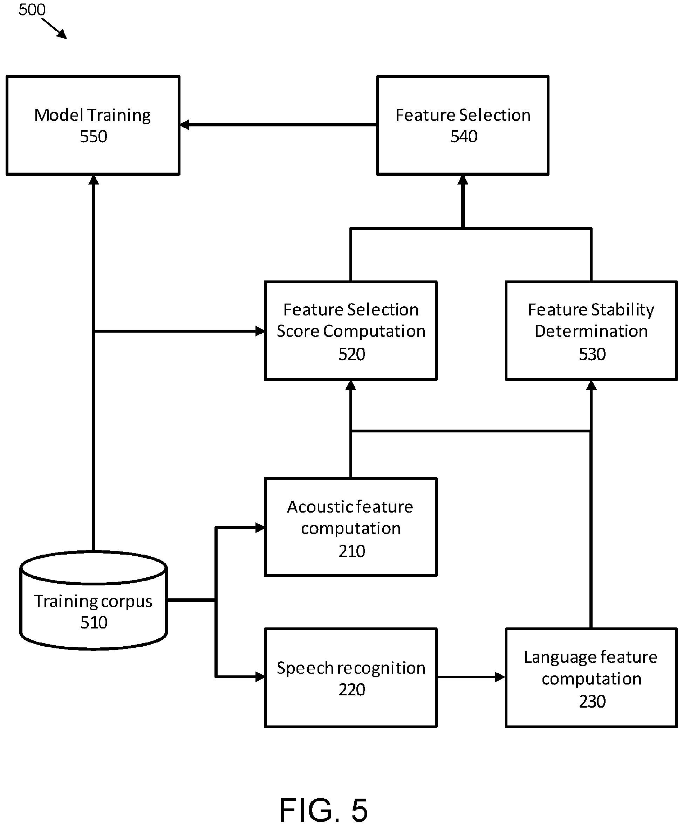

Figure 5 is an exemplary system 500 that may be used to select features for training a mathematical model for diagnosing a medical condition, and then using the selected features to train the mathematical model. System 500 may be used multiple times to select features for different medical conditions. For example, a first use of system 500 may select features for diagnosing concussions and a second use of system 500 may select features for diagnosing Alzheimer's disease.

Figure 5 includes a training corpus 510 of speech data items for training a mathematical model for diagnosing a medical condition. Training corpus 510 may include any appropriate information, such as speech data of multiple people with and without the medical condition, a label indicating whether or not person has the medical condition, and any other information described herein.

Acoustic feature computation component 210, speech recognition component 220, and language feature computation component 230 may be implemented as described above to compute acoustic and language features for the speech data in the training corpus. Acoustic feature computation component 210 and language feature computation component 230 may compute a large number of features so that the best performing features may be determined. This may be in contrast to Figure 2 where these components are used in a production system and thus these components may compute only the features that were previously selected.

Feature selection score computation component 520 may compute a selection score for each feature (which may be an acoustic feature, a language feature, or any other feature described herein). To compute a selection score for a feature, a pair of numbers may be created for each speech data item in the training corpus, where the first number of the pair is the value of the feature, and the second number of the pair is an indicator of the medical condition diagnosis. The value for the indicator of the medical condition diagnosis may have two values (e.g., 0 if the person does not have the medical condition and 1 if the person has the medical condition) or may have a larger number of values (e.g., a real number between 0 and 1 or multiple integers indicating a likelihood or severity of the medical condition).

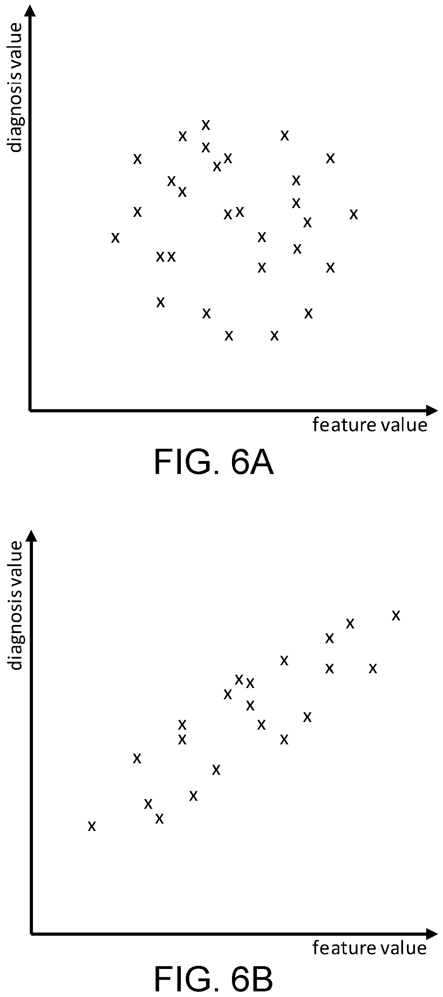

Accordingly, for each feature, a pair of numbers may be obtained for each speech data item of the training corpus. Figs. 6A and 6B illustrate two conceptual plots of the pairs of numbers for a first feature and a second feature. For Figure 6A, there does not appear to be a pattern or correlation between the values of the first feature and the corresponding diagnosis values, but for Figure 6B, there does appear to be a pattern or correlation between the values of the second feature and the diagnosis values. Accordingly, one may conclude that the second feature is likely a useful feature for determining whether a person has the medical condition and that the first feature is not.

Feature selection score computation component 520 may compute a selection score for a feature using the pairs of feature values and diagnosis values. Feature selection score computation component 520 may compute any appropriate score that indicates a pattern or correlation between the feature values and the diagnosis values. For example, feature selection score computation component 520 may compute a Rand index, an adjusted Rand index, mutual information, adjusted mutual information, a Pearson correlation, an absolute Pearson correlation, a Spearman correlation, or an absolute Spearman correlation.

The selection score may indicate the usefulness of the feature in detecting a medical condition. For example, a high selection score may indicate that a feature should be used in training the mathematical model, and a low selection score may indicate that the feature should not be used in training the mathematical model.

Feature stability determination component 530 may determine if a feature (which may be an acoustic feature, a language feature, or any other feature described herein) is stable or unstable. To make a stability determination, the speech data items may be divided into multiple groups, which may be referred to as folds. For example, the speech data items may be divided into five folds. In some implementations, the speech data items may be divided into folds such that each fold has an approximately equal number of speech data items for different genders and age groups.

The statistics of each fold may be compared to statistics of the other folds. For example, for a first fold, the median (or mean or any other statistic relating to the center or middle of a distribution) feature value (denoted as M₁) may be determined. Statistics may also be computed for the combination of the other folds. For example, for the combination of the other folds, the median of the feature values (denoted as Mₒ) and a statistic measuring of variability of the feature values (denoted as Vₒ), such as interquartile range, variance, or standard deviation, may be computed. The feature may be determined to be unstable if the median of the first fold differs too greatly from the median of the second fold. For example, the feature may be determined to be unstable if: ${M}_{1} \left〈{M}_{o}-C\frac{{V}_{o}}{2} or {M}_{1}\right〉 {M}_{o} + C \frac{{V}_{o}}{2}$ where C is a scaling factor. The process may then be repeated for each of the other folds. For example, the median of a second fold may be compared with median and variability of the other folds as described above.

In some implementations, if, after comparing each fold to the other folds, the median of each fold is not too far from the median of the other folds, then the feature may be determined to be stable. Conversely, if the median of any fold is too far from the median of the other folds, then the feature may be determined to be unstable.

In some implementations, feature stability determination component 530 may output a Boolean value for each feature to indicate whether the feature is stable or not. In some implementations, stability determination component 530 may output a stability score for each feature. For example, a stability score may be computed as the largest distance between the median of a fold and the other folds (e.g., a Mahalanobis distance).

Feature selection component 540 may receive the selection scores from feature selection score computation component 520 and the stability determinations from feature stability determination component 530 and select a subset of features to be used to train the mathematical model. Feature selection component 540 may select several features having the highest selection scores that are also sufficiently stable.

In some implementations, the number of features to be selected (or a maximum number of features to be selected) may be set ahead of time. For example, a number N may be determined based on the amount of training data, and N features may be selected. The selected features may be determined by removing unstable features (e.g., features determined to be unstable or features with a stability score below a threshold) and then selecting the N features with the highest selection scores.

In some implementations, the number of features to be selected may be based on the selection scores and stability determinations. For example, the selected features may be determined by removing unstable features, and then selecting all features with a selection score above a threshold.

In some implementations, the selection scores and stability scores may be combined when selecting features. For example, for each feature a combined score may be computed (such as by adding or multiplying the selection score and the stability score for the feature) and features may be selected using the combined score.

Model training component 550 may then train a mathematical model using the selected features. For example, model training component 550 may iterate over the speech data items of the training corpus, obtain the selected features for the speech data items, and then train the mathematical model using the selected features. In some implementations, dimension reduction techniques, such as principal components analysis or linear discriminant analysis, may be applied to the selected features as part of the model training. Any appropriate mathematical model may be trained, such as any of the mathematical models described herein.

In some implementations, other techniques, such as wrapper methods, may be used for feature selection or may be used in combination with the feature selection techniques presented above. Wrapper methods may select a set of features, train a mathematical model using the selected set of features, and then evaluate the performance of the set of features using the trained model. Where the number of possible features is relatively small and/or training time is relatively short, all possible sets of features may be evaluated, and the best performing set may be selected. Where the number of possible features is relatively large and/or the training time is a significant factor, optimization techniques may be used to iteratively find a set of features that performs well. **In** some implementations, a set of features may be selected using system 500, and then a subset of these features may be selected using wrapper methods as the final set of features.

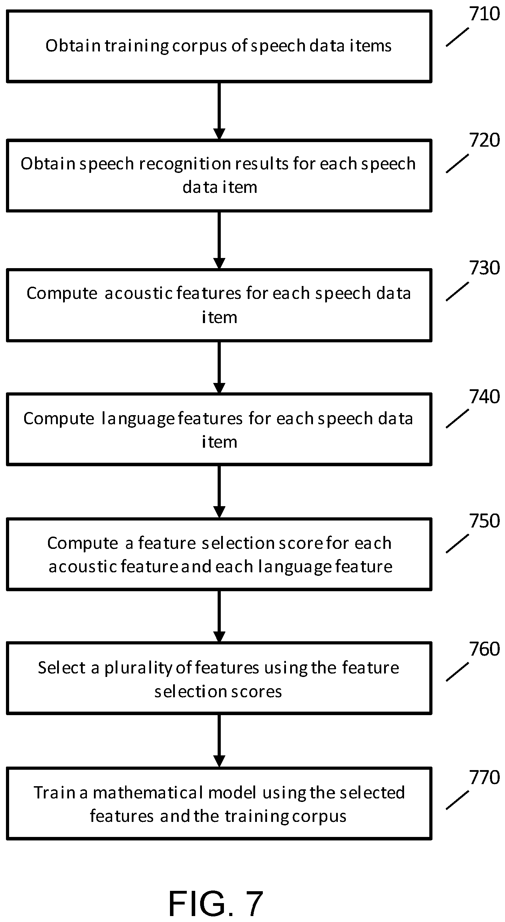

Figure 7 is a flowchart of an example implementation of selecting features for training a mathematical model for diagnosing a medical condition. In Figure 7 and other flowcharts herein, the ordering of the steps is exemplary and other orders are possible, not all steps are required, steps may be combined (in whole or part) or subdivided and, in some implementations, some steps may be omitted, or other steps may be added. The methods described by any flowcharts described herein may be implemented, for example, by any of the computers or systems described herein.

At step 710, a training corpus of speech data items is obtained. The training corpus may include a representation of an audio signal of a person's speech, an indication of a medical diagnosis of the person from whom the speech was obtained, and any other appropriate information, such as any of the information described herein.

At step 720, speech recognition results are obtained for each speech data item of the training corpus. The speech recognition results may have been computed in advance and stored with the training corpus or stored in another location. The speech recognition results may include any appropriate information, such as a transcript, a list of highest scoring transcripts (e.g., an N-best list), a lattice of possible transcriptions, and timing information, such as the start and end time of words, pause fillers, or other speech units.

At step 730, acoustic features are computed for each speech data item of the training corpus. Acoustic features may include any features that are computed without using speech recognition results of a speech data item, such as any of the acoustic features described herein. Acoustic features may include or be computed from data used in the speech recognition process (e.g., mel-frequency cepstral coefficients or perceptual linear predictors), but acoustic features do not use speech recognition results, such as information about the words or pause fillers present in a speech data item.

At step 740, language features are computed for each speech data item of the training corpus. Language features may include any features that are computed using speech recognition results, such as any of the language features described herein.

At step 750, a feature selection score is computed for each acoustic feature and each language feature. To compute a feature selection score for the feature, the value of the feature for each speech data item in the training corpus may be used along with other information, such as a known diagnosis value corresponding to the speech data item. The feature selection score may be computed using any of the techniques described herein, such as by computing an absolute Pearson correlation. In some implementations, feature selection scores may be computed for other features as well, such as features relating to demographic information of a person.

At step 760, a plurality of features is selected using the feature selection scores. For example, a number of features having the highest selection scores may be selected. In some implementations, a stability determination may be computed for each feature and the plurality of features may be selected using both the feature selection scores and the stability determinations, such as by using any of the techniques described herein.

At step 770, a mathematical model is trained using the selected features. Any appropriate mathematical model may be trained, such as a neural network or a support vector machine. After the mathematical model has been trained, it may be deployed in a production system, such as a diagnostic apparatus 104, a system 109 of Figure 1B, or the like to perform diagnosis of medical conditions.

The steps of Figure 7 may be performed in a variety of manners. For example, in some implementations, steps 730, and 740 may be performed in a loop that loops over each of the speech data items in the training corpus. For a first iteration, acoustic and language features may be computed for a first speech data item, for a second iteration, acoustic and language features may be computed for a second speech data item, and so forth.

When using a deployed model for diagnosing a medical condition, the person being diagnosed may be presented with a sequence of prompts or questions to obtain speech from the person. Any appropriate prompts may be used, such as any of the prompts of Figure 4. After the features have been selected, as described above, prompts may be selected so that the selected prompts provide useful information about the selected features.

For example, suppose that a selected feature is pitch. While pitch has been determined to be a useful feature for diagnosing a medical condition, some prompts may be better than others in obtaining a useful pitch feature. Very short utterances (e.g., yes/no answers) may not provide sufficient data to accurately compute pitch and thus prompts that generate longer responses may be more useful in obtaining information about pitch.

For another example, suppose that a selected feature is word difficulty. While word difficulty has been determined to be a useful feature for diagnosing a medical condition, some prompts may be better than others in obtaining a useful word difficulty feature. Prompts that ask a user to read a presented passage will generally result in speech of the words in the passage, and thus the word difficulty feature would have the same value each time this prompt is presented, and thus this prompt would not be useful in obtaining information about word difficulty. By contrast, open ended questions, such as "Tell me about your day?", may result in greater variability of vocabulary in responses and thus may provide more useful information about word difficulty.

Selecting a set of prompts may also improve the performance of a system for diagnosing medical conditions and provide a better experience for the person being evaluated. By using the same set of prompts for each person being evaluated, the system for diagnosing medical conditions may provide more accurate results, since the data collected from multiple people may be more comparable than if different prompts were used with each person. Further, using a defined set of prompts allows the evaluation of a person to be more predictable and of a desired duration that is appropriate for the evaluation of the medical condition. For example, for evaluating whether a person has Alzheimer's disease, it may be acceptable to use more prompts to collect a larger amount of data, but for evaluating whether a person has a concussion during a sporting event, it may be necessary to use a smaller number of prompts to obtain a result more quickly.

In some implementations, prompts may be selected by computing prompt selection scores. A training corpus may have multiple or even many speech data items for a single prompt. For example, the training corpus may include examples of the prompt used with different people or the same prompt may be used with the same person multiple times.

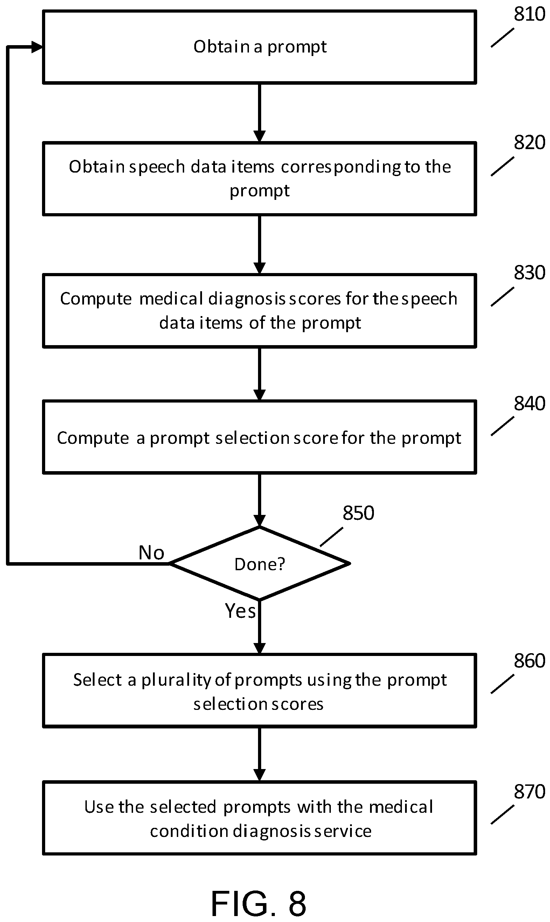

Figure 8 is a flowchart of an example implementation of selecting prompts for use with a deployed model for diagnosing a medical condition.

Steps 810 to 840 may be performed for each prompt (or a subset of the prompts) in the training corpus to compute a prompt selection score for each prompt.

At step 810 a prompt is obtained, and at step 820 speech data items corresponding to the prompt are obtained from the training corpus.

At step 830, a medical diagnosis score is computed for each speech data item corresponding to the prompt. For example, a medical diagnosis score for a speech data item may be a number output by a mathematical model (e.g., the mathematical model trained in Figure 7) indicating a likelihood that a person has the medical condition and/or a severity of the medical condition.

At step 840, a prompt selection score is computed for the prompt using the computed medical diagnosis scores. The computation of a prompt selection score may be similar to the computation of a feature selection score, as described above. For each speech data item corresponding to the prompt, a pair of numbers may be obtained. For each pair, the first number of the pair may be the computed medical diagnosis score computed from the speech data item, and the second number of the pair may be a known medical condition diagnosis of the person (e.g., the person is known to have the medical condition or a severity of the medical condition). Plotting these pairs of numbers may result in a plot similar to Figure 6A or Figure 6B, and depending on the prompt there may or may not be a pattern or correlation in the pairs of numbers.

A prompt selection score for a prompt may include any score that indicates a pattern or correlation between the computed medical diagnosis scores and the known medical condition diagnoses. For example, a prompt selection score may include a Rand index, an adjusted Rand index, mutual information, adjusted mutual information, a Pearson correlation, an absolute Pearson correlation, a Spearman correlation, or an absolute Spearman correlation.

At step 850 it is determined if other prompts remain to be processed. If prompts remain to be processed, then processing may proceed to step 810 to process additional prompts. If all prompts have been processed, then processing may proceed to step 860.

At step 860, a plurality of prompts are selected using the prompt selection scores. For example, a number of prompts having the highest prompt selection scores may be selected. In some implementations, a stability determination may be computed for each prompt and the plurality of prompts may be selected using both the prompt selection scores and the prompt stability determinations, such as by using any of the techniques described herein.

At step 870, the selected prompts are used with a deployed medical condition diagnosis service. For example, when diagnosing a person, the selected prompts may be presented to a person to obtain speech of the person in response to each of the prompts.

In some implementations, other techniques, such as wrapper methods, may be used for prompt selection or may be used in combination with the prompt selection techniques presented above. In some implementations, a set of prompts may be selected using the process of Figure 8, and then a subset of these prompts may be selected using wrapper methods as the final set of features.

In some implementations, a person involved with creating the medical condition diagnosis service may assist in the selection of prompts. The person may use his knowledge or experience to select prompts based on the selected features. For example, where a selected feature is word difficulty, the person may review the prompts and select prompts that are more likely to provide useful information relating to word difficulty. The person may select one or more prompts that are likely to provide useful information for each of the selected features.

In some implementations, the person may review the prompts selected by the process of Figure 8 and add or remove prompts to improve the performance of a medical condition diagnosis system. For example, two prompts may each provide useful information about word difficulty, but the information provided by the two prompts may be largely redundant, and using both prompts may not provide significant benefit over using just one of them.

In some implementations, a second mathematical model may be trained after prompt selection that is adapted to the selected prompts. The mathematical model trained in Figure 7 may process a single utterance (in response to a prompt) to generate a medical diagnosis score. Where the process of performing a diagnosis comprises processing multiple utterances corresponding to multiple prompts, then each of the utterances may be processed by the mathematical model of Figure 7 to generate multiple medical diagnosis scores. To determine an overall medical diagnosis, the multiple medical diagnosis scores may need to be combined in some way. Accordingly, the mathematical model trained in Figure 7 may not be adapted to a selected set of prompts.

When the selected prompts are used in a session to diagnose a person, each of the prompts may be presented to the person to obtain an utterance corresponding to each of the prompts. Instead of processing the utterances separately, the utterances may be processed simultaneously by the model to generate a medical diagnosis score. Accordingly, a model may be adapted to the selected prompts because it is trained to simultaneously process utterances corresponding to each of the selected prompts.

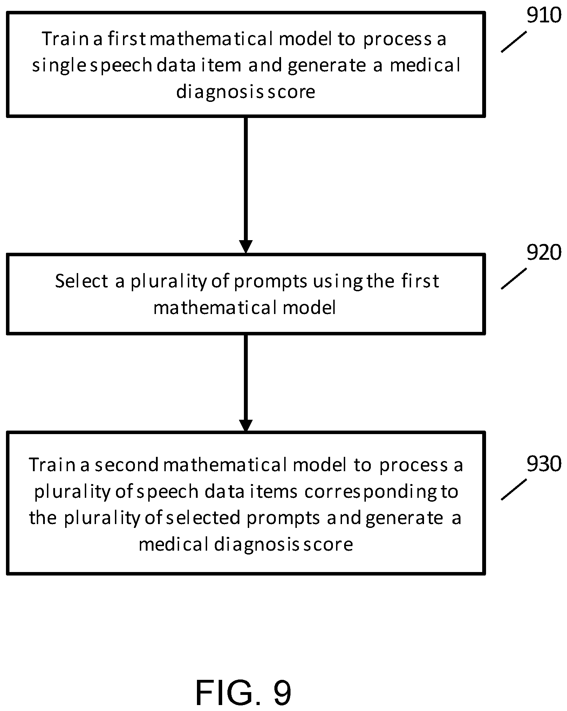

Figure 9 is a flowchart of an example implementation training a mathematical model that is adapted to a set of selected prompts. At step 910, a first mathematical model is obtained, such as by using the process of Figure 7. At step 920, a plurality of prompts are selected using the first mathematical model, such as by the process of Figure 8.

At step 930, a second mathematical model is trained that simultaneously processes multiple speech data items corresponding to the plurality of selected prompts to generate a medical diagnosis score. When training the second mathematical model, a training corpus may be used that includes sessions with speech data items corresponding to each of the plurality of selected prompts. When training the mathematical model, the input to the mathematical model may be fixed to the speech data items from the session and corresponding to each of the selected prompts. The output of the mathematical model may be fixed to a known medical diagnosis. The parameters of the model may then be trained to optimally process the speech data item simultaneously to generate a medical diagnosis score. Any appropriate training techniques may be used, such as stochastic gradient descent.

The second mathematical model may then be deployed as part of a medical condition diagnosis service, such as a diagnostic apparatus 104, the service of Figure 1, or the like. The second mathematical model may provide better performance than the first mathematical model because it has been trained to process the utterances simultaneously rather than individual and thus the training may be better able to combine the information from all the of utterances to generate the medical condition diagnosis score.

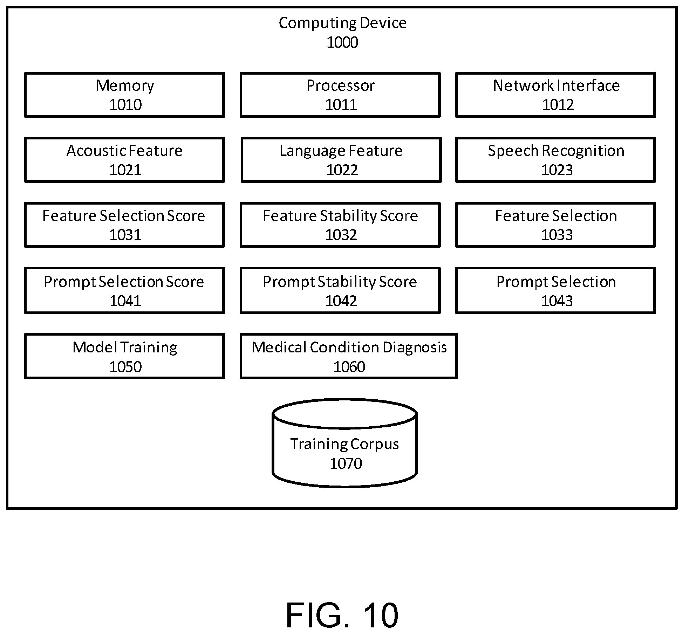

Figure 10 illustrates components of one implementation of a computing device 1000 for implementing any of the techniques described above. In Figure 10, the components are shown as being on a single computing device, but the components may be distributed among multiple computing devices, such as a system of computing devices, including, for example, an end-user computing device (e.g., a smart phone or a tablet) and/or a server computing device (e.g., cloud computing).

Computing device 1000 may include any components typical of a computing device, such as volatile or nonvolatile memory 1010, one or more processors 1011, and one or more network interfaces 1012. Computing device 1000 may also include any input and output components, such as displays, keyboards, and touch screens. Computing device 1000 may also include a variety of components or modules providing specific functionality, and these components or modules may be implemented in software, hardware, or a combination thereof. Below, several examples of components are described for one example implementation, and other implementations may include additional components or exclude some of the components described below.

Computing device 1000 may have an acoustic feature computation component 1021 that may compute acoustic features for a speech data item as described above. Computing device 1000 may have a language feature computation component 1022 that may compute language features for a speech data item as described above. Computing device 1000 may have a speech recognition component 1023 that may generate speech recognition results for a speech data item as described above. Computing device 1000 may have a feature selection score computation component 1031 that may compute selection scores for features as described above. Computing device 1000 may have a feature stability score computation component 1032 that may make stability determinations or compute stability scores as described above. Computing device 1000 may have a feature selection component 1033 that may select features using selection scores and/or stability determinations as described above. Computing device 1000 may have a prompt selection score computation component 1041 that may compute selection scores for prompts as described above. Computing device 1000 may have a prompt stability score computation component 1042 that may make stability determinations or compute stability scores as described above. Computing device 1000 may have a prompt selection component 1043 that may select prompts using selection scores and/or stability determinations as described above. Computing device 1000 may have a model training component 1050 that may train mathematical models as described above. Computing device 1000 may have a medical condition diagnosis component 1060 that may process speech data items to determine a medical diagnosis score as described above.

Computing device 1000 may include or have access to various data stores, such as training corpus data store 1070. Data stores may use any known storage technology such as files, relational or non-relational databases, or any non-transitory computer-readable media.

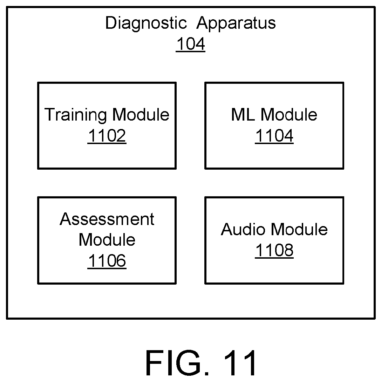

Figure 11 depicts one embodiment of a diagnostic apparatus 104 for techniques for speech language model training and application. The diagnostic apparatus 104, in certain embodiments, may be substantially similar to one or more of a device diagnostic apparatus 104a and/or a backend diagnostic apparatus 104b, as described above with regard to Figure 1A. The diagnostic apparatus 104, in the depicted embodiment, a training module 1102, an ML module 1104, an assessment module 1106, and an audio module 1108, which are described in more detail below.

In one embodiment, the training module 1102 is configured to train a first speech model in a first language. A speech model, as used herein, may refer to a machine learning model that is trained to analyze, forecast, predict, process, or the like speech data, e.g., audio data of a user's voice when speaking. An example of a speech model may be an x-vector embedding speech model. X-vector models may be fixed-length representations of variable-length speech segments. They are embeddings extracted from a deep neural network (DNN) that takes vectors as input. X-vectors are known to capture speaker characteristics, even when the speakers have not been seen during the DNN training. However, x-vectors are just one example of a speech model that may be used. One of skill in the art would recognize other speech models that may be utilized.

In one embodiment, the first speech model is used to determine one or more characteristics of speech data that is indicative of MCI, or another medical condition. The speech data, for example, may include voice or audio data from a user, or a plurality of users, where at least a subset of the users have MCI, or another medical condition. The training module 1102 may train the first speech model on speech data that is captured or associated with a first language, e.g., English, French, German, Japanese, or the like.

In such an embodiment, the first speech model may be trained to analyze sublanguage characteristics of the speech data. The sublanguage characteristics, for instance, may include acoustic features that include any features computed from the speech data that do not involve or depend on performing speech recognition on the speech data (e.g., the acoustic features do not use information about the words spoken in the speech data). For example, acoustic features may include mel-frequency cepstral coefficients, perceptual linear prediction features, jitter, shimmer, speech tone, speech rate, speech patterns, and/or the like.

The characteristics may include language features that are computed using the results of a speech recognition. For example, language features may include a speaking rate (e.g., the number of vowels or syllables per second), a number of pause fillers (e.g., "ums" and "ahs"), the difficulty of words (e.g., less common words), or the parts of speech of words following pause fillers.

In one embodiment, the training module 1102 may access a training corpus, as described above with reference to Figures 3 and 4, that includes speech data for training the first speech model. For example, the training corpus may include database entries where each entry includes an identifier of a person, the known diagnosis of the person (e.g., no MCI, MCI, or the like), an identifier of a prompt or question that was presented to a person (e.g., "How are you today?"), a filename of a file that contains the speech data, and/or a link to the file that contains the speech data.

In one embodiment, the training module 1102 is configured to further train the first speech model on non-speech data for assessing MCI. In such an embodiment, when an assessment is performed, the user's non-speech data is provided to the first speech model to determine the assessment of MCI.

Non-speech data, as used herein, may refer to other data that is captured and may be indicative of one or more symptoms of MCI. For instance, the non-speech data may include data that is captured from one or more sensors associated with the user, from images or videos of the user, from descriptive information about the user, and/or the like. The training module 1102 may receive the non-speech training data from a data store or data base, from a remote location, from a website, and/or the like.

In one embodiment, the non-speech data includes demographic information such as gender, age, weight, height, place of birth or residence, and/or the like. The ML module 1104, described below, may receive the demographic information from the user (e.g., in response to a prompt), from public records (e.g., publicly accessible records or data available online), from a user profile, from social media, and/or the like.

In one embodiment, the non-speech data includes motion data, such as gait data. As used herein, gait data may refer to data that describes the manner in which the user walks, jogs, runs, or the like. In one embodiment, the ML module 1104may receive gait data for a user from data captured from motion sensors such as an accelerometer, a gyroscope, and/or the like, e.g., from a user's device.

In one embodiment, the non-speech data includes activity data. Activity data may refer to data that describes different activities that the user performs throughout the day, e.g., brushing teeth, making breakfast, exercising, eating, sleeping, or the like. The activity data may include sensor data, as described above, but may also include audio, image, or video data captured using audio-visual cameras, e.g., located in or around the user's home, which the ML module 1104 may interact or communicate with to receive the activity data for a user.

In one embodiment, the non-speech data includes driving-related data associated with the user's driving history. Driving-related data may refer to data that indicates that the user is authorized to drive, data that describes how the user drives, how often the user drives, where the user drives, the length (in distance and/or time) that the user drives, driving accident information, and/or the like. In such an embodiment, the ML module 1104 may receive driving-related data from a user (e.g., self-reported information), may access public records such as DMV databases (e.g., via an API, a screen scrape, or the like) to determine whether the user has a valid driver's license, to determine accident information for the user, and/or the like. Other driving data may be captured by sensors associated with the user, e.g., GPS sensors or accelerometers to determine the user's driving locations, speeds, distances, or the like.

In one embodiment, the non-speech data includes medication information. The medication information may include types of medications that the user takes, how often the user takes the medications, the side effects of the medications, the dosages of the medications, whether the medications require a prescription or are available over-the-counter, the last time the user took medications, and/or the like. In one embodiment, the ML module 1104 may receive the medication information from a user (e.g., self-reported), from tracked or monitored activity data (e.g., camera or video data that shows the user taking medications

In one embodiment, the non-speech data includes motor function data describing one or more motor functions for a user. Motor function data, as used herein, may refer to data that describes a user's ability to perform tasks that require certain movements of the user's body, e.g., data describing the types of tasks the user can perform (e.g., changing a light bulb, replacing batteries in a device, opening a jar, or the like), the amount of time it takes to complete certain tasks, and/or the like. The ML module 1104 may receive the motor function data for a user from the user, from a device associated with the user, from camera or video content, and/or the like.

In one embodiment, the training module 1102 is configured to train a second speech model for use in a second language (different from the first language) using at least a portion of the first speech model trained in the first language. For instance, if the first speech model is trained on speech data associated with English, the first speech model may be used to further train a second speech model for a different language such as Japanese. In such an embodiment, at least a portion of the first speech model may be used to train the second speech model, may be used as a base model for the second speech model (e.g., which can further be refined or trained using speech data from the second language), and/or the like. Even though the languages that are being analyzed are different, the first speech model may act as a base or training model for the second speech model due to similarities in sublanguage characteristics between different languages.

In one embodiment, the training module 1102 may employ transfer learning to train the second speech model using at least a portion of the first speech model. Transfer learning, as used herein, may refer to applying knowledge of an already trained machine learning model to a different machine learning model, e.g., for a different task. For instance, applying knowledge gained from the first speech model that is trained in the first language to detect MCI to a second speech model that is trained in a different language to detect MCI. In this manner, using transfer learning allows for efficiencies in speech model training, especially where training data is not available or is deficient, lacking, or incomplete.

In one embodiment, the ML module 1104 is configured to apply the second speech model that is trained for use in the second language to speech data for a user that is captured in the second language. In such an embodiment, the ML module 1104 may receive speech data in the second language and provide the received speech data as input into the second speech model (which has been trained at least in part using the first speech model for a different language).

The ML module 1104 may receive the speech data from a user, from a database or other data store, and/or the like. For instance, the ML module 1104 may be in communication with a database or data store and may access the speech data for a user from the database based on one or more parameters, variables, or the like (e.g., based on a date range, a type of data (e.g., speech and/or non-speech data), and/or the like.

As described above with regard to the acoustic feature computation component 210, the speech recognition component 220, the language feature computation component 230, and/or the medical condition classifier 240, in certain embodiments, the ML module 1104 may extract one or more voice features (e.g., acoustic features and/or language features) from a voice recording (e.g., baseline response data and/or test case response data) and may input the one or more extracted voice features into the second speech model, which may output information that the assessment module 1106 can use to assess the likelihood that the user has a medical condition such as MCI.

In a further embodiment, in addition to inputting extracted voice features into the second speech model to diagnose a medical condition, the ML module 1104 may input other supplemental data associated with the user into the model, such as the non-speech data described above, which the assessment module 1106 may use to diagnose the medical condition based on the result. For example, the ML module 1104 may input sensor data from a computing device 102 of a user into a model (e.g., together with extracted voice features or other voice data), camera data, video data, driving-related data, medication data, activity data, demographic data, motor-function data, and/or the like to determine an assessment or other diagnoses of a medical condition such as MCI for a user.

In one embodiment, the ML module 1104 may extract one or more image features from image data (e.g., one or more images, video, or the like of the user, of the user's face, of another body part of the user associated with a medical condition, or the like) from an image sensor such as a camera of a computing device 102, and may input the one or more image features into a model (e.g., with extracted voice features or the like). In a further embodiment, the ML module 1104 may base an assessment or other diagnosis at least partially on touch inputs received from a user on a touchscreen, touchpad, or the like of a computing device 102.

In certain embodiments, the ML module 1104 may be configured to provide output, using the second speech model, for making an assessment or other diagnosis of a medical condition based on one or more acoustic features of received speech data, without regard to one or more language features of the received speech data, e.g., based on sublanguage characteristics of the speech, without any language features, with only one or more predefined language features, without any automatic speech recognition, or the like.

In one embodiment, the assessment module 1106 is configured to determine, based on output from the second speech model that is trained for use in the second language, an assessment of MCI for the user. The assessment may include the probability or likelihood that the user has MCI, a prognosis for the user, treatment recommendations, and/or the like.

In this manner, in some embodiments, the assessment and/or diagnosis of the output of the second speech model may be independent of a language and/or a dialect of the received verbal response, so the assessment module 1106 may provide assessments and/or diagnoses for users in different languages using acoustic features of the received speech data and/or the non-speech data. In other words, a speech model that has been trained using data in a first language such as English can be used to train or refine a different speech model for a different language such as Japanese and provide accurate results that can be used to assess a user's likelihood of having MCI or another medical condition.

In one embodiment, the audio module 1108 is configured to capture and process speech data that is received from the user. In such an embodiment, the audio module 1108 may receive a plurality of different recorded audio clips of the user's speech and create a single recorded audio clip of the user's speech from a plurality of shorter audio clips of the user's verbal responses to at least one query by combining the plurality of shorter audio clips into a single audio clip having a length that satisfies a threshold length, e.g., thirty seconds, a minute, or the like. In certain embodiments, the audio module 1108 may record the user's verbal responses to an unprompted dialog (or monolog), e.g., such as a general discussion between two or more parties where the dialog is not overtly prompting speech (which may be captured using sensors of a user's device, an IoT device, and/or the like).

In such an embodiment, the audio module 1108 questions and/or queries a user with multiple questions, prompts, requests, or the like to elicit a plurality of different responses. The audio module 1108, in certain embodiments, may audibly and/or verbally question a user (e.g., using a speaker of a computing device 102 such as an integrated speaker, headphones, a Bluetooth^{®} speaker or headphones, or the like). For example, due to certain potential medical conditions, such as MCI, it may be difficult for a user to read a question and/or prompt, and audibly questioning the user may simplify and/or expedite a diagnosis.

In a further embodiment, the audio module 1108 may display one or more questions and/or other prompts to a user (e.g., on an electronic display screen of a computing device 102, or the like), another user (e.g., a caretaker, a parent, a medical professional, an administrator, or the like) may read one or more questions and/or other prompts to a user, or the like. In various embodiments, the one or more questions or prompts may be selected as described above with regard to the prompt selection component 1043, or the like, in order to facilitate a diagnosis of one or more medical conditions.

In certain embodiments, a plurality of audio modules 1108 disposed on a plurality of different computing devices 102 may query and/or question a plurality of different users. For example, a plurality of distributed audio modules 1108 may collect voice samples for a medical trial, to train a speech model for diagnosing a medical condition, to collect test data to facilitate prompt selection, or the like.

The audio module 1108, in one embodiment, questions and/or otherwise queries a user at a predefined health state, such as a known healthy state, a predefined stage of a medical condition, or the like, to collect one or more baseline voice recordings, training data, or other data. In certain embodiments, the audio module 1108 questions and/or otherwise queries a user in response to a potential medical event or other trigger.

The audio module 1108 may question a user in order to collect test case voice recordings or other test case data in response to a user requesting a medical assessment, based on data from a sensor of a computing device 102 such as a wearable or mobile device, and/or based on receiving another trigger indicating that an injury may have occurred, that one or more symptoms of a disease have been detected, or the like.

In certain embodiments, the audio module 1108 may question and/or prompt a user about general information, tasks that the user is performing, activities that the user has participated in, the user's daily activities, and/or the like to elicit responses from the user that can be recorded and analyzed.

For example, the audio module 1108 may audibly and/or textually question the user "what did you do today?", "what did you eat today?", "did you drive today?", "did you take your medications today?", "what medications do you take?", "do you have trouble with daily tasks?", "what is the date today?", "what is the day of the week?", "what year is it?", "what time is it right now?", may audibly list words and/or numbers for the user and ask the user to repeat them back, may display a series of pictures to the user and ask the user to repeat back a description of the series of pictures, or the like.

In one embodiment, the audio module 1108 is configured to receive response data (e.g., voice data of a verbal response), in response to one or more questions and/or other queries from the audio module 1108. For example, in certain embodiments, the audio module 1108 may use a microphone of a computing device 102, e.g., a smart phone, to record verbal responses (e.g., answers) of a user to one or more questions or other prompts from the audio module 1108.

In one embodiment, the audio module 1108 may present a prompt to the user to elicit a longer, free-form response. For instance, the prompt may be "Tell me about your day today," where the user is expected to provide a response that is longer than a single word answer or a yes/no answer. In one embodiment, if the audio module 1108 detects that the user is done providing a response, the audio module 1108 may present one or more follow-up prompts or questions to continue the conversation. In this manner, the audio module 1108 can receive conversational speech data from the user that includes or demonstrates various language and sublanguage characteristics, which the audio module 1108 can process and analyze to select snippets from the different responses and combine the snippets into a single audio clip.

The audio module 1108, in one embodiment, may store received response data such as voice recordings or the like on a computer readable storage medium of a computing device 102, 110, so that the detection module 1106 may access and/or process the received response data to provide the data to a speech model to diagnose and/or assess a medical condition, train a speech model for diagnosing and/or assessing a medical condition, or the like. In another embodiment, the audio module 1108 may provide received response data directly to the detection module 1106 for diagnosing and/or assessing a medical condition (e.g., without otherwise storing the data, temporarily storing and/or caching the data, or the like).

In one embodiment, the audio module 1108 is configured to remove audio clips from the user that are shorter than a predefined or threshold length. For instance, the audio module 1108 may remove responses that are one word, that are shorter than a threshold length (e.g., ten words) or the like. In further embodiments, the audio module 1108 may remove responses that are shorter than a predefined or threshold duration, e.g., shorter than three seconds, or the like. In this manner, the audio module 1108 can filter out outliers that may not include substantive data to contribute to training a speech model, or that is otherwise usable for providing an assessment of the medical condition, e.g., MCI.

In one embodiment, the audio module 1108 is configured to remove audio clips of the plurality of shorter audio clips that do not demonstrate predefined speech characteristics. In one embodiment, the predefined speech characteristics may include a number of words, a number of syllables, a pronunciation, a tone, acoustic speech features, prosodic speech features, linguistic speech features, a signal-to-noise ratio, a length of speech, and/or the like. In such an embodiment, the audio module 1108 may flag certain audio clips or snippets for removal from the set of shorter audio clips, so that they are not included in the combined audio clip, if the flagged audio clips do not demonstrate certain speech characteristics, e.g., sublanguage characteristics, that are useful for assessing whether a user has a medical condition such as MCI.

In one example embodiment, the audio module 1108 may capture verbal or speech responses from users during a phone conversation, a phone survey, or the like. During the phone survey, the caller may use a predefined script that includes questionnaires and example questions to elicit free speech from the participants. The free speech questions may include aspects of daily life, shopping, housework, jobs, or the like.

In one embodiment, the audio module 1108 captures the audio clips or files in a certain format, such as an uncompressed WAV format to eliminate potential negative effect of compression on the final prediction of our models. Since the audio is captured via conversations over the phone - including potentially band-limiting landline, Bluetooth, or recording devices - the audio module 1108 re-samples recordings to ensure a consistent sampling rate, e.g., re-sampling to 8 kHz.

In one embodiment, the audio module 1108 performs an initial segmentation by checking pause and speech durations. Further, the audio module 1108 filters out audio segments that are not representative of the medical condition, e.g., MCI. The audio module 1108, in one embodiment, measures the duration of voice activity by summing the duration of speech segments - determined by an automatic speech recognition (ASR) system - excluding pauses between words. The audio module 1108 may remove short audio samples based on the voice activity duration. The audio module 1108 concatenates the resulting audio clips/files in one audio sample per phone call.

The training module 1102 and/or the ML module 1104, in one embodiment, converts the variable-length audio input signal to a fixed-sized representation, e.g., a feature vector embodied as an x-vector. Based on the representation, in one embodiment, the training module 1102 builds models at two different levels: first, per-speaker models, and second, per-segment models. For the per-speaker models, a single feature vector is generated given a segment of per-speaker (concatenated) audio. In one embodiment, a fully-connected DNN architecture may be applied. For the per-segment model, each speaker's audio input is split into a set of fixed-length segments. Based on findings of preliminary experiments, the segment length is fixed to 5 seconds and is created every 2.5 seconds. Each segment is treated as an independent sample, and the model is trained to return the corresponding label.

In one embodiment, in each model type the final layer is a probability layer that returns two probability values, one for Normal and one for a has-MCI class indicator (both summing up to 1). To determine the final prediction given a speaker in the per-segment model, in one embodiment, the assessment module 1106 averages the output prediction probabilities for the has-MCI class for all segments whose probability is greater than the 5 percentile and lower than the 95 percentile per speaker. If the average is greater than or equal to 0.51, the final prediction is determined as has-MCI. In the per-speaker model, the final prediction label for a given speaker is determined by averaging each segment's prediction output.

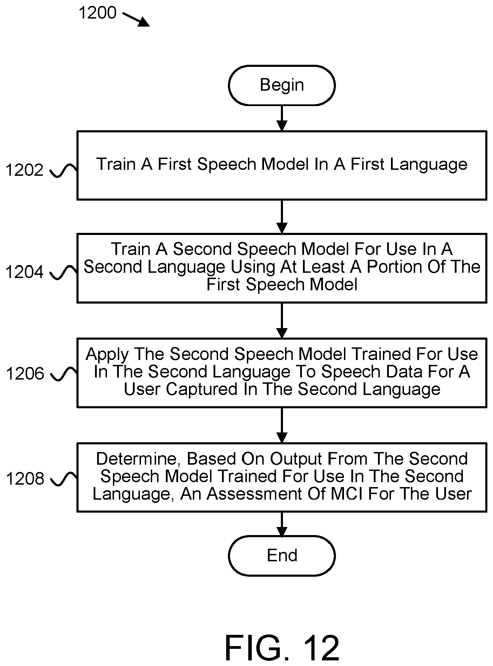

Figure 12 depicts one embodiment of a method 1200 for techniques for speech language model training and application. In one embodiment, the method 1200 is performed by a computing device 102, a diagnostic apparatus 104, a device diagnostic apparatus 104a, a backend diagnostic apparatus 104b, a training module 1102, an ML module 1104, an assessment module 1106, an audio module 1108, a mobile computing device 102, a backend server computing device 108, and/or the like.

The method 1200 begins, and trains 1202 a first speech model in a first language, the first speech model used to determine one or more characteristics of speech data that is indicative of MCI. The method 1200 trains 1204 a second speech model for use in a second language using at least a portion of the first speech model trained in the first language.

The method 1200 applies 1206 the second speech model trained for use in the second language to speech data for a user captured in the second language. The method 1200 determines 1208, based on output from the second speech model trained for use in the second language, an assessment of MCI for the user, and the method 1200 ends.

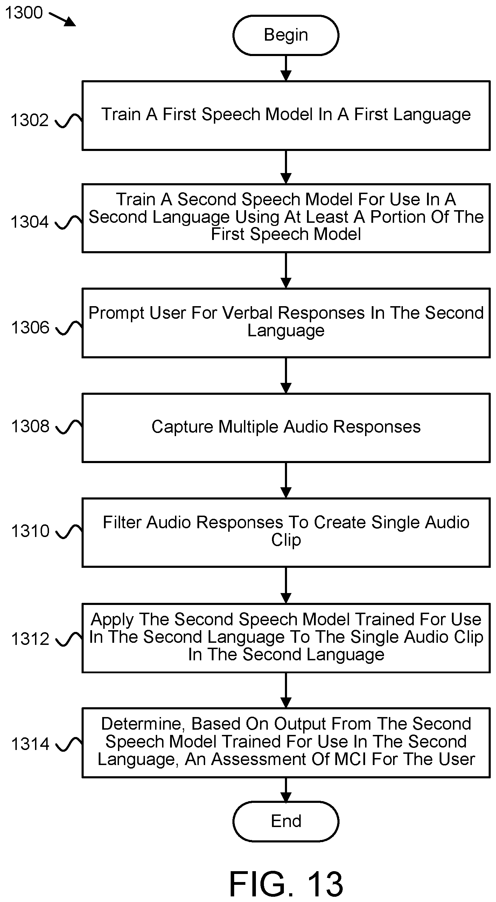

Figure 13 depicts one embodiment of a method 1300 for techniques for speech language model training and application. In one embodiment, the method 1300 is performed by a computing device 102, a diagnostic apparatus 104, a device diagnostic apparatus 104a, a backend diagnostic apparatus 104b, a training module 1102, an ML module 1104, an assessment module 1106, an audio module 1108, a mobile computing device 102, a backend server computing device 108, and/or the like.

The method 1300 begins, and trains 1302 a first speech model in a first language, the first speech model used to determine one or more characteristics of speech data that is indicative of MCI. The method 1300 trains 1304 a second speech model for use in a second language using at least a portion of the first speech model trained in the first language, e.g., via transfer learning.

In one embodiment, the method 1300 prompts 1306 a user for verbal responses in the second language, captures 1308 multiple audio responses from the user, e.g., multiple audio clips, and filters 1310 the audio responses to create a single audio clip. In such an embodiment, the method 1300 may filter out audio clips that are not a threshold direction or length, that do not include desired language characteristics, or the like.

The method 1300 applies 1312 the second speech model trained for use in the second language to speech data for a user captured in the second language. The method 1300 determines 1314, based on output from the second speech model trained for use in the second language, an assessment of MCI for the user, and the method 1300 ends.

A means for training a first speech model in a first language that is used to determine one or more characteristics of speech data that is indicative of MCI, in various embodiments, may comprise a diagnostic apparatus 104, a device diagnostic apparatus 104a, a backend diagnostic apparatus 104b, a training module 1102, a mobile computing device 102, a backend server computing device 108, an electronic speaker of a computing device 102, 108, headphones, an electronic display screen of a computing device 102, 108, a user interface device, a network interface, a mobile application, a processor, an application specific integrated circuit (ASIC), a field programmable gate array (FPGA), programmable logic, other logic hardware, and/or other executable program code stored on a non-transitory computer readable storage medium. Other embodiments may comprise substantially similar or equivalent means for training a first speech model in a first language.

A means for training a second speech model for use in a second language using at least a portion of the first speech model trained in the first language, in various embodiments, may comprise a diagnostic apparatus 104, a device diagnostic apparatus 104a, a backend diagnostic apparatus 104b, a training module 1102, a mobile computing device 102, a backend server computing device 108, an electronic speaker of a computing device 102, 108, headphones, an electronic display screen of a computing device 102, 108, a user interface device, a network interface, a mobile application, a processor, an application specific integrated circuit (ASIC), a field programmable gate array (FPGA), programmable logic, other logic hardware, and/or other executable program code stored on a non-transitory computer readable storage medium. Other embodiments may comprise substantially similar or equivalent means for training a second speech model in a second language.

A means for applying the second speech model trained for use in the second language to speech data for a user captured in the second language, in various embodiments, may comprise a diagnostic apparatus 104, a device diagnostic apparatus 104a, a backend diagnostic apparatus 104b, an ML module 1104, a mobile computing device 102, a backend server computing device 108, a mobile application, machine learning, artificial intelligence, an acoustic feature computation component 210, a speech recognition component 220, a Gaussian mixture model, an acoustic model, a language model, a neural network, a deep neural network, a medical condition classifier 240, a classifier, a support vector machine, a multi-layer perceptron, a processor, an application specific integrated circuit (ASIC), a field programmable gate array (FPGA), programmable logic, other logic hardware, and/or other executable program code stored on a non-transitory computer readable storage medium. Other embodiments may comprise substantially similar or equivalent means for applying the second speech model.

A means for determining, based on output from the second speech model trained for use in the second language, an assessment of MCI for the user, in various embodiments, may comprise a diagnostic apparatus 104, a device diagnostic apparatus 104a, a backend diagnostic apparatus 104b, an assessment module 1106, a mobile computing device 102, a backend server computing device 108, a mobile application, a processor, an application specific integrated circuit (ASIC), a field programmable gate array (FPGA), programmable logic, other logic hardware, and/or other executable program code stored on a non-transitory computer readable storage medium. Other embodiments may comprise substantially similar or equivalent means for determining an assessment of MCI.

A means for creating a recorded audio clip from a plurality of shorter audio clips, in various embodiments, may comprise a diagnostic apparatus 104, a device diagnostic apparatus 104a, a backend diagnostic apparatus 104b, an audio module 1108, a mobile computing device 102, a backend server computing device 108, a mobile application, a processor, an application specific integrated circuit (ASIC), a field programmable gate array (FPGA), programmable logic, other logic hardware, and/or other executable program code stored on a non-transitory computer readable storage medium. Other embodiments may comprise substantially similar or equivalent means for creating a recorded audio clip from a plurality of shorter audio clips.

The methods and systems described herein may be deployed in part or in whole through a machine that executes computer software, program codes, and/or instructions on a processor. "Processor" as used herein is meant to include at least one processor and unless context clearly indicates otherwise, the plural and the singular should be understood to be interchangeable. Any aspects of the present disclosure may be implemented as a method on the machine, as a system or apparatus as part of or in relation to the machine, or as a computer program product embodied in a computer readable medium executing on one or more of the machines. The processor may be part of a server, client, network infrastructure, mobile computing platform, stationary computing platform, or other computing platform.

A processor may be any kind of computational or processing device capable of executing program instructions, codes, binary instructions, and the like. The processor may be or include a signal processor, digital processor, embedded processor, microprocessor, or any variant such as a co-processor (math co-processor, graphic co-processor, communication co-processor and the like) and the like that may directly or indirectly facilitate execution of program code or program instructions stored thereon. In addition, the processor may enable execution of multiple programs, threads, and codes. The threads may be executed simultaneously to enhance the performance of the processor and to facilitate simultaneous operations of the application. By way of implementation, methods, program codes, program instructions and the like described herein may be implemented in one or more thread. The thread may spawn other threads that may have assigned priorities associated with them; the processor may execute these threads based on priority or any other order based on instructions provided in the program code. The processor may include memory that stores methods, codes, instructions, and programs as described herein and elsewhere. The processor may access a storage medium through an interface that may store methods, codes, and instructions as described herein and elsewhere. The storage medium associated with the processor for storing methods, programs, codes, program instructions or other type of instructions capable of being executed by the computing or processing device may include but may not be limited to one or more of a CD-ROM, DVD, memory, hard disk, flash drive, RAM, ROM, cache and the like.

A processor may include one or more cores that may enhance speed and performance of a multiprocessor. In embodiments, the process may be a dual core processor, quad core processors, other chip-level multiprocessor and the like that combine two or more independent cores (called a die).

The methods and systems described herein may be deployed in part or in whole through a machine that executes computer software on a server, client, firewall, gateway, hub, router, or other such computer and/or networking hardware. The software program may be associated with a server that may include a file server, print server, domain server, internet server, intranet server and other variants such as secondary server, host server, distributed server, and the like. The server may include one or more of memories, processors, computer readable media, storage media, ports (physical and virtual), communication devices, and interfaces capable of accessing other servers, clients, machines, and devices through a wired or a wireless medium, and the like. The methods, programs, or codes as described herein and elsewhere may be executed by the server. In addition, other devices required for execution of methods as described in this application may be considered as a part of the infrastructure associated with the server.

The server may provide an interface to other devices including, without limitation, clients, other servers, printers, database servers, print servers, file servers, communication servers, distributed servers, and the like. Additionally, this coupling and/or connection may facilitate remote execution of program across the network. The networking of some or all these devices may facilitate parallel processing of a program or method at one or more locations without deviating from the scope of the disclosure. **In** addition, any of the devices attached to the server through an interface may include at least one storage medium capable of storing methods, programs, code and/or instructions. A central repository may provide program instructions to be executed on different devices. In this implementation, the remote repository may act as a storage medium for program code, instructions, and programs.

The software program may be associated with a client that may include a file client, print client, domain client, internet client, intranet client and other variants such as secondary client, host client, distributed client, and the like. The client may include one or more of memories, processors, computer readable media, storage media, ports (physical and virtual), communication devices, and interfaces capable of accessing other clients, servers, machines, and devices through a wired or a wireless medium, and the like. The methods, programs, or codes as described herein and elsewhere may be executed by the client. In addition, other devices required for execution of methods as described in this application may be considered as a part of the infrastructure associated with the client.

The client may provide an interface to other devices including, without limitation, servers, other clients, printers, database servers, print servers, file servers, communication servers, distributed servers, and the like. Additionally, this coupling and/or connection may facilitate remote execution of program across the network. The networking of some or all these devices may facilitate parallel processing of a program or method at one or more locations without deviating from the scope of the disclosure. **In** addition, any of the devices attached to the client through an interface may include at least one storage medium capable of storing methods, programs, applications, code and/or instructions. A central repository may provide program instructions to be executed on different devices. In this implementation, the remote repository may act as a storage medium for program code, instructions, and programs.

The methods and systems described herein may be deployed in part or in whole through network infrastructures. The network infrastructure may include elements such as computing devices, servers, routers, hubs, firewalls, clients, personal computers, communication devices, routing devices and other active and passive devices, modules and/or components as known in the art. The computing and/or non-computing device(s) associated with the network infrastructure may include, apart from other components, a storage medium such as flash memory, buffer, stack, RAM, ROM and the like. The processes, methods, program codes, instructions described herein and elsewhere may be executed by one or more of the network infrastructural elements.

The methods, program codes, and instructions described herein and elsewhere may be implemented on a cellular network having multiple cells. The cellular network may either be frequency division multiple access (FDMA) network or code division multiple access (CDMA) network. The cellular network may include mobile devices, cell sites, base stations, repeaters, antennas, towers, and the like. The cell network may be a GSM, GPRS, 3G, EVDO, mesh, or other network types.

The methods, programs codes, and instructions described herein and elsewhere may be implemented on or through mobile devices. The mobile devices may include navigation devices, cell phones, mobile phones, mobile personal digital assistants, laptops, palmtops, netbooks, pagers, electronic books readers, music players and the like. These devices may include, apart from other components, a storage medium such as a flash memory, buffer, RAM, ROM and one or more computing devices. The computing devices associated with mobile devices may be enabled to execute program codes, methods, and instructions stored thereon. Alternatively, the mobile devices may be configured to execute instructions in collaboration with other devices. The mobile devices may communicate with base stations interfaced with servers and configured to execute program codes. The mobile devices may communicate on a peer-to-peer network, mesh network, or other communications network. The program code may be stored on the storage medium associated with the server and executed by a computing device embedded within the server. The base station may include a computing device and a storage medium. The storage device may store program codes and instructions executed by the computing devices associated with the base station.

The computer software, program codes, and/or instructions may be stored and/or accessed on machine readable media that may include: computer components, devices, and recording media that retain digital data used for computing for some interval of time; semiconductor storage known as random access memory (RAM); mass storage typically for more permanent storage, such as optical discs, forms of magnetic storage like hard disks, tapes, drums, cards and other types; processor registers, cache memory, volatile memory, non-volatile memory; optical storage such as CD, DVD; removable media such as flash memory (e.g. USB sticks or keys), floppy disks, magnetic tape, paper tape, punch cards, standalone RAM disks, Zip drives, removable mass storage, off-line, and the like; other computer memory such as dynamic memory, static memory, read/write storage, mutable storage, read only, random access, sequential access, location addressable, file addressable, content addressable, network attached storage, storage area network, bar codes, magnetic ink, and the like.

The methods and systems described herein may transform physical and/or intangible items from one state to another. The methods and systems described herein may also transform data representing physical and/or intangible items from one state to another.

Reference throughout this specification to "one embodiment," "an embodiment," or similar language means that a particular feature, structure, or characteristic described in connection with the embodiment is included in at least one embodiment. Thus, appearances of the phrases "in one embodiment," "in an embodiment," and similar language throughout this specification may, but do not necessarily, all refer to the same embodiment, but mean "one or more but not all embodiments" unless expressly specified otherwise. The terms "including," "comprising," "having," and variations thereof mean "including but not limited to" unless expressly specified otherwise. An enumerated listing of items does not imply that any or all of the items are mutually exclusive and/or mutually inclusive, unless expressly specified otherwise. The terms "a," "an," and "the" also refer to "one or more" unless expressly specified otherwise.

Furthermore, the described features, advantages, and characteristics of the embodiments may be combined in any suitable manner. One skilled in the relevant art will recognize that the embodiments may be practiced without one or more of the specific features or advantages of a particular embodiment. In other instances, additional features and advantages may be recognized in certain embodiments that may not be present in all embodiments.

These features and advantages of the embodiments will become more fully apparent from the following description and appended claims or may be learned by the practice of embodiments as set forth hereinafter. As will be appreciated by one skilled in the art, aspects of the present invention may be embodied as a system, method, and/or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module," or "system." Furthermore, aspects of the present invention may take the form of a computer program product embodied in one or more computer readable medium(s) having program code embodied thereon.

Many of the functional units described in this specification have been labeled as modules (or components), to more particularly emphasize their implementation independence. For example, a module may be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

Modules may also be implemented in software for execution by various types of processors. An identified module of program code may, for instance, comprise one or more physical or logical blocks of computer instructions which may, for instance, be organized as an object, procedure, or function. Nevertheless, the executables of an identified module need not be physically located together but may comprise disparate instructions stored in different locations which, when joined logically together, comprise the module and achieve the stated purpose for the module.

Indeed, a module of program code may be a single instruction, or many instructions, and may even be distributed over several different code segments, among different programs, and across several memory devices. Similarly, operational data may be identified and illustrated herein within modules and may be embodied in any suitable form and organized within any suitable type of data structure. The operational data may be collected as a single data set or may be distributed over different locations including over different storage devices, and may exist, at least partially, merely as electronic signals on a system or network. Where a module or portions of a module are implemented in software, the program code may be stored and/or propagated on in one or more computer readable medium(s).

The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory ("RAM"), a read-only memory ("ROM"), an erasable programmable read-only memory ("EPROM" or Flash memory), a static random access memory ("SRAM"), a portable compact disc read-only memory ("CD-ROM"), a digital versatile disk ("DVD"), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. **In** the latter scenario, the remote computer may be connected to the user's computer through any type of network, including a local area network (LAN) or a wide area network (WAN), or the connection may be made to an external computer (for example, through the Internet using an Internet Service Provider). **In** some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The schematic flowchart diagrams and/or schematic block diagrams in the Figures illustrate the architecture, functionality, and operation of possible implementations of apparatuses, systems, methods and computer program products according to various embodiments of the present invention. In this regard, each block in the schematic flowchart diagrams and/or schematic block diagrams may represent a module, segment, or portion of code, which comprises one or more executable instructions of the program code for implementing the specified logical function(s).

It should also be noted that, in some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. Other steps and methods may be conceived that are equivalent in function, logic, or effect to one or more blocks, or portions thereof, of the illustrated Figures.

Although various arrow types and line types may be employed in the flowchart and/or block diagrams, they are understood not to limit the scope of the corresponding embodiments. Indeed, some arrows or other connectors may be used to indicate only the logical flow of the depicted embodiment. For instance, an arrow may indicate a waiting or monitoring period of unspecified duration between enumerated steps of the depicted embodiment. It will also be noted that each block of the block diagrams and/or flowchart diagrams, and combinations of blocks in the block diagrams and/or flowchart diagrams, can be implemented by special purpose hardware-based systems that perform the specified functions or acts, or combinations of special purpose hardware and program code.

The present invention may be embodied in other specific forms. The scope of the invention is, therefore, indicated by the appended claims rather than by the foregoing description.

## Claims

1. An apparatus, comprising:
a processor; and
a memory coupled with the processor, the memory storing code that is executable by the processor to cause the apparatus to:
train a first speech model in a first language, the first speech model being trained on (i) speech data in the first language and (ii) non-speech data associated with each speaker, the trained first speech model used to determine one or more characteristics of speech data that is indicative of mild cognitive impairment, MCI;
train a second speech model for use in a second language using at least a portion of the first speech model trained in the first language;
apply the second speech model trained for use in the second language to (i) speech data for a user captured in the second language and (ii) non-speech data for the user; and
determine, based on output from the second speech model trained for use in the second language, an assessment of MCI for the user.

2. The apparatus of claim 1, wherein the first speech model trained in the first language is configured to analyze sub language characteristics of the speech data.

3. The apparatus of claim 2, wherein the sub language characteristics comprise at least one of a speech tone, a speech rate, a speech pattern, acoustic speech characteristics, prosodic speech characteristics, and linguistic speech features.

4. The apparatus of any preceding claim, wherein the user's non-speech data further provided to the first speech model trained in the first language to determine the assessment of MCI for the user.

5. The apparatus of claim 4, wherein the non-speech data comprises at least one of:
(i) demographic information for the user;
(ii) gait data, the gait data describing the user's manner of walking;
(iii) activity data, the activity data captured from one or more sensors associated with the user;
(iv) driving-related data associated with the user's driving history;
(v) medication information for the user; and/or
(vi) data describing one or more motor functions for the user.

6. The apparatus of any preceding claim, wherein the speech data comprises a recorded audio clip of verbal responses by the user in response to at least one of at least one query and unprompted dialog.

7. The apparatus of claim 6, wherein the code is further executable by the processor to create the recorded audio clip from a plurality of shorter audio clips of the user's verbal responses to the at least one query by combining the plurality of shorter audio clips into a single audio clip having a length that satisfies a threshold length.

8. The apparatus of claim 7, wherein the code is further executable by the processor to present multiple prompts to the user to elicit the plurality of shorter audio clips.

9. The apparatus of claim 7 or 8, wherein the plurality of audio clips comprise snippets taken of a longer conversation that demonstrate various predefined language characteristics.

10. The apparatus of any one of claims 7 to 9, wherein the code is further executable by the processor to remove audio clips of the plurality of shorter audio clips that are shorter than a threshold length.

11. The apparatus of any one of claims 7 to 10, wherein the code is further executable by the processor to remove audio clips of the plurality of shorter audio clips that do not demonstrate predefined speech characteristics.

12. The apparatus of claim 11, wherein the predefined speech characteristics comprise at least one of a number of words, a number of syllables, a pronunciation, a tone, a signal-to-noise ratio, and a length of speech.

13. The apparatus of any preceding claim, wherein the first speech model, the second speech model, or a combination thereof comprises an x-vector embedding speech model.

14. A method, comprising:
training a first speech model in a first language, the first speech model being trained on (i) speech data in the first language and (ii) non-speech data associated with each speaker, the trained first speech model used to determine one or more characteristics of speech data that is indicative of mild cognitive impairment, MCI;
training a second speech model for use in a second language using at least a portion of the first speech model trained in the first language;
applying the second speech model trained for use in the second language to (i) speech data for a user captured in the second language and (ii) non-speech data for the user; and
determining, based on output from the second speech model trained for use in the second language, an assessment of MCI for the user.

15. An apparatus, comprising:
means for training a first speech model in a first language, the first speech model being trained on (i) speech data in the first language and (ii) non-speech data associated with each speaker, the trained first speech model used to determine one or more characteristics of speech data that is indicative of mild cognitive impairment, MCI;
means for training a second speech model for use in a second language using at least a portion of the first speech model trained in the first language;
means for applying the second speech model trained for use in the second language to (i) speech data for a user captured in the second language and (ii) non-speech data for the user; and
means for determining, based on output from the second speech model trained for use in the second language, an assessment of MCI for the user.

## Patentansprüche

1. Gerät, umfassend:
einen Prozessor; und
einen mit dem Prozessor gekoppelten Speicher, wobei der Speicher Code speichert, der durch den Prozessor ausführbar ist, um das Gerät zu Folgendem zu veranlassen:
Trainieren eines ersten Sprachmodells in einer ersten Sprache, wobei das erste Sprachmodell anhand von (i) Sprachdaten in der ersten Sprache und (ii) Nicht-Sprachdaten, die jedem Sprecher zugeordnet sind, trainiert wird, wobei das trainierte erste Sprachmodell verwendet wird, um ein oder mehrere Merkmale von Sprachdaten zu bestimmen, die auf eine leichte kognitive Beeinträchtigung, MCI, hinweisen;
Trainieren eines zweiten Sprachmodells zur Verwendung in einer zweiten Sprache unter Verwendung mindestens eines Abschnitts des in der ersten Sprache trainierten ersten Sprachmodells;
Anwenden des zur Verwendung in der zweiten Sprache trainierten zweiten Sprachmodells auf (i) Sprachdaten für einen Benutzer, die in der zweiten Sprache erfasst wurden, und (ii) Nicht-Sprachdaten für den Benutzer; und Bestimmen, auf der Grundlage einer Ausgabe des zur Verwendung in der zweiten Sprache trainierten zweiten Sprachmodells, einer Bewertung der MCI für den Benutzer.

2. Gerät nach Anspruch 1, wobei das in der ersten Sprache trainierte erste Sprachmodell konfiguriert ist, um subsprachliche Merkmale der Sprachdaten zu analysieren.

3. Gerät nach Anspruch 2, wobei die subsprachlichen Merkmale mindestens eines von einem Sprachton, einer Sprechgeschwindigkeit, einem Sprachmuster, akustischen Sprachmerkmalen, prosodischen Sprachmerkmalen und linguistischen Spracheigenschaften umfassen.

4. Gerät nach einem der vorstehenden Ansprüche, wobei die Nicht-Sprachdaten des Benutzers ferner dem in der ersten Sprache trainierten ersten Sprachmodell bereitgestellt werden, um die Bewertung der MCI für den Benutzer zu bestimmen.

5. Gerät nach Anspruch 4, wobei die Nicht-Sprachdaten mindestens eines der Folgenden umfassen:
(i) demografische Informationen für den Benutzer;
(ii) Gangdaten, wobei die Gangdaten die Art und Weise des Gehens des Benutzers beschreiben;
(iii) Aktivitätsdaten, wobei die Aktivitätsdaten von einem oder mehreren dem Benutzer zugeordneten Sensoren erfasst werden;
(iv) fahrbezogene Daten, die der Fahrhistorie des Benutzers zugeordnet sind;
(v) Arzneimittelinformationen für den Benutzer; und/oder
(vi) Daten, die eine oder mehrere motorische Funktionen des Benutzers beschreiben.

6. Gerät nach einem der vorstehenden Ansprüche, wobei die Sprachdaten einen aufgezeichneten Audioclip verbaler Antworten des Benutzers als Reaktion auf mindestens eines von mindestens einer Abfrage und einem unaufgeforderten Dialog umfassen.

7. Gerät nach Anspruch 6, wobei der Code ferner durch den Prozessor ausführbar ist, um den aufgezeichneten Audioclip aus einer Vielzahl von kürzeren Audioclips der verbalen Antworten des Benutzers auf die mindestens eine Abfrage zu erstellen, indem die Vielzahl von kürzeren Audioclips zu einem einzelnen Audioclip kombiniert wird, der eine Länge aufweist, die eine Schwellenlänge erfüllt.

8. Gerät nach Anspruch 7, wobei der Code ferner durch den Prozessor ausführbar ist, um dem Benutzer mehrere Aufforderungen zu präsentieren, um die Vielzahl von kürzeren Audioclips hervorzurufen.

9. Gerät nach Anspruch 7 oder 8, wobei die Vielzahl von Audioclips Ausschnitte umfasst, die aus einem längeren Gespräch entnommen wurden und verschiedene vordefinierte sprachliche Merkmale aufweisen.

10. Gerät nach einem der Ansprüche 7 bis 9, wobei der Code ferner durch den Prozessor ausführbar ist, um aus der Vielzahl von kürzeren Audioclips Audioclips zu entfernen, die kürzer als eine Schwellenlänge sind.

11. Gerät nach einem der Ansprüche 7 bis 10, wobei der Code ferner durch den Prozessor ausführbar ist, um aus der Vielzahl von kürzeren Audioclips Audioclips zu entfernen, die keine vordefinierten Sprachmerkmale aufweisen.

12. Gerät nach Anspruch 11, wobei die vordefinierten Sprachmerkmale mindestens eines von einer Anzahl von Wörtern, einer Anzahl von Silben, einer Aussprache, einem Ton, einem Signal-Rausch-Verhältnis und einer Sprachlänge umfassen.

13. Gerät nach einem der vorstehenden Ansprüche, wobei das erste Sprachmodell, das zweite Sprachmodell oder eine Kombination davon ein x-Vektor-Einbettungs-Sprachmodell umfasst.

14. Verfahren, umfassend:
Trainieren eines ersten Sprachmodells in einer ersten Sprache, wobei das erste Sprachmodell anhand von (i) Sprachdaten in der ersten Sprache und (ii) Nicht-Sprachdaten, die jedem Sprecher zugeordnet sind, trainiert wird, wobei das trainierte erste Sprachmodell verwendet wird, um ein oder mehrere Merkmale von Sprachdaten zu bestimmen, die auf eine leichte kognitive Beeinträchtigung, MCI, hinweisen;
Trainieren eines zweiten Sprachmodells zur Verwendung in einer zweiten Sprache unter Verwendung mindestens eines Abschnitts des in der ersten Sprache trainierten ersten Sprachmodells;
Anwenden des zur Verwendung in der zweiten Sprache trainierten zweiten Sprachmodells auf (i) Sprachdaten für einen Benutzer, die in der zweiten Sprache erfasst wurden, und (ii) Nicht-Sprachdaten für den Benutzer; und
Bestimmen, auf der Grundlage einer Ausgabe des zur Verwendung in der zweiten Sprache trainierten zweiten Sprachmodells, einer Bewertung der MCI für den Benutzer.

15. Gerät, umfassend:
Mittel zum Trainieren eines ersten Sprachmodells in einer ersten Sprache, wobei das erste Sprachmodell anhand von (i) Sprachdaten in der ersten Sprache und (ii) Nicht-Sprachdaten, die jedem Sprecher zugeordnet sind, trainiert wird, wobei das trainierte erste Sprachmodell verwendet wird, um ein oder mehrere Merkmale von Sprachdaten zu bestimmen, die auf eine leichte kognitive Beeinträchtigung, MCI, hinweisen;
Mittel zum Trainieren eines zweiten Sprachmodells zur Verwendung in einer zweiten Sprache unter Verwendung mindestens eines Abschnitts des in der ersten Sprache trainierten ersten Sprachmodells;
Mittel zum Anwenden des zur Verwendung in der zweiten Sprache trainierten zweiten Sprachmodells auf (i) Sprachdaten für einen Benutzer, die in der zweiten Sprache erfasst wurden, und (ii) Nicht-Sprachdaten für den Benutzer; und
Mittel zum Bestimmen, auf der Grundlage einer Ausgabe des zur Verwendung in der zweiten Sprache trainierten zweiten Sprachmodells, einer Bewertung der MCI für den Benutzer.

## Revendications

1. Appareil comprenant :
un processeur ; et
une mémoire couplée au processeur, la mémoire stockant un code exécutable par le processeur pour amener l'appareil à :
entraîner un premier modèle vocal dans une première langue, le premier modèle vocal étant entraîné sur (i) des données vocales dans la première langue et (ii) des données non vocales associées à chaque locuteur, le premier modèle vocal entraîné étant utilisé pour déterminer une ou plusieurs caractéristiques de données vocales qui sont indicatives d'un trouble cognitif léger, MCI ;
entraîner un second modèle vocal destiné à être utilisé dans une seconde langue en utilisant au moins une partie du premier modèle vocal entraîné dans la première langue ;
appliquer le second modèle vocal entraîné pour être utilisé dans la seconde langue à (i) des données vocales pour un utilisateur capturées dans la seconde langue et (ii) des données non vocales pour l'utilisateur ; et déterminer, sur la base d'une sortie du second modèle vocal entraîné pour être utilisé dans la seconde langue, une évaluation du MCI pour l'utilisateur.

2. Appareil selon la revendication 1, dans lequel le premier modèle vocal entraîné dans la première langue est configuré pour analyser des caractéristiques sous-linguistiques des données vocales.

3. Appareil selon la revendication 2, dans lequel les caractéristiques sous-linguistiques comprennent au moins l'un parmi un ton de parole, un débit de parole, un motif de parole, des caractéristiques acoustiques de parole, des caractéristiques prosodiques de parole et des caractéristiques linguistiques de parole.

4. Appareil selon l'une quelconque des revendications précédentes, dans lequel les données non vocales de l'utilisateur sont en outre fournies au premier modèle vocal entraîné dans la première langue pour déterminer l'évaluation du MCI pour l'utilisateur.

5. Appareil selon la revendication 4, dans lequel les données non vocales comprennent au moins l'un parmi :
(i) des informations démographiques pour l'utilisateur ;
(ii) des données de démarche, les données de démarche décrivant la manière de marcher de l'utilisateur ;
(iii) des données d'activité, les données d'activité étant capturées à partir d'un ou plusieurs capteurs associés à l'utilisateur ;
(iv) des données relatives à la conduite associées à l'historique de conduite de l'utilisateur ;
(v) des informations de médication pour l'utilisateur ; et/ou
(vi) des données décrivant une ou plusieurs fonctions motrices pour l'utilisateur.

6. Appareil selon l'une quelconque des revendications précédentes, dans lequel les données vocales comprennent un extrait audio enregistré de réponses verbales formulées par l'utilisateur en réponse à au moins l'une parmi au moins une question et une conversation spontanée.

7. Appareil selon la revendication 6, dans lequel le code est en outre exécutable par le processeur pour créer l'extrait audio enregistré à partir d'une pluralité d'extraits audio plus courts de réponses verbales de l'utilisateur à l'au moins une question en combinant la pluralité d'extraits audio plus courts en un seul extrait audio ayant une longueur qui satisfait une longueur seuil.

8. Appareil selon la revendication 7, dans lequel le code est en outre exécutable par le processeur pour présenter de multiples invites à l'utilisateur afin d'obtenir la pluralité d'extraits audio plus courts.

9. Appareil selon la revendication 7 ou 8, dans lequel la pluralité d'extraits audio comprend des fragments tirés d'une conversation plus longue qui démontrent diverses caractéristiques linguistiques prédéfinies.

10. Appareil selon l'une quelconque des revendications 7 à 9, dans lequel le code est en outre exécutable par le processeur pour supprimer des extraits audio de la pluralité d'extraits audio plus courts qui sont plus courts qu'une longueur seuil.

11. Appareil selon l'une quelconque des revendications 7 à 10, dans lequel le code est en outre exécutable par le processeur pour supprimer des extraits audio de la pluralité d'extraits audio plus courts qui ne démontrent pas de caractéristiques de parole prédéfinies.

12. Appareil selon la revendication 11, dans lequel les caractéristiques de parole prédéfinies comprennent au moins l'un parmi un nombre de mots, un nombre de syllabes, une prononciation, un ton, un rapport signal/bruit et une longueur de parole.

13. Appareil selon l'une quelconque des revendications précédentes, dans lequel le premier modèle vocal, le second modèle vocal ou une combinaison de ceux-ci comprend un modèle vocal d'incorporation par vecteur x.

14. Procédé, comprenant :
l'entraînement d'un premier modèle vocal dans une première langue, le premier modèle vocal étant entraîné sur (i) des données vocales dans la première langue et (ii) des données non vocales associées à chaque locuteur, le premier modèle vocal entraîné étant utilisé pour déterminer une ou plusieurs caractéristiques de données vocales qui sont indicatives d'un trouble cognitif léger, MCI ;
l'entraînement d'un second modèle vocal destiné à être utilisé dans une seconde langue en utilisant au moins une partie du premier modèle vocal entraîné dans la première langue ;
l'application du second modèle vocal entraîné pour être utilisé dans la seconde langue à (i) des données vocales pour un utilisateur capturées dans la seconde langue et (ii) des données non vocales pour l'utilisateur ; et
la détermination, sur la base d'une sortie du second modèle vocal entraîné pour être utilisé dans la seconde langue, d'une évaluation du MCI pour l'utilisateur.

15. Appareil comprenant :
des moyens pour entraîner un premier modèle vocal dans une première langue, le premier modèle vocal étant entraîné sur (i) des données vocales dans la première langue et (ii) des données non vocales associées à chaque locuteur, le premier modèle vocal entraîné étant utilisé pour déterminer une ou plusieurs caractéristiques de données vocales qui sont indicatives d'un trouble cognitif léger, MCI ;
des moyens pour entraîner un second modèle vocal destiné à être utilisé dans une seconde langue en utilisant au moins une partie du premier modèle vocal entraîné dans la première langue ;
des moyens pour appliquer le second modèle vocal entraîné pour être utilisé dans la seconde langue à (i) des données vocales pour un utilisateur capturées dans la seconde langue et (ii) des données non vocales pour l'utilisateur ; et
des moyens pour déterminer, sur la base d'une sortie du second modèle vocal entraîné pour être utilisé dans la seconde langue, une évaluation du MCI pour l'utilisateur.
